# EUROPEAN PATENT SPECIFICATION

(11) **EP 1 239 876 B1**
(45) Date of publication and mention of the grant of the patent: **30.07.2008**
(21) Application number: 00975681.8
(22) Date of filing: 17.11.2000
(51) Int. Cl.: A61K 39/385, A61K 39/29, A61P 31/12

(54) **HCV VACCINE COMPOSITIONS**
HCV-IMPFSTOFF ZUSAMMENSETZUNGEN
COMPOSITIONS VACCINALES HCV

(30) Priority: 19.11.1999 US 166652 P; 11.08.2000 US 224362 P
(43) Date of publication of application: 18.09.2002
(73) Proprietor: CSL Limited, Parkville, VIC 3052 (AU); Novartis Vaccines and Diagnostics, Inc., Emeryville, CA 94608 (US)
(72) Inventor: DRANE, Debbie, Bullengarook, VIC 3437 (AU); COX, John, Bullengarook, VIC 3437 (AU); HOUGHTON, Michael, Novartis Vaccines and Diagnostics, Inc., Emeryville, CA 94608 (US); PALIARD, Xavier, Novartis Vaccines and Diagnostics, Inc., Emeryville, CA 94608 (US)
(74) Representative: Duckworth, Timothy John
(86) International application number: PCT/AU2000/001410
(87) International publication number: WO 2001/037869

(56) References cited:
- WO-A-00/48630
- WO-A-90/01947
- WO-A-96/04385
- WO-A-97/01640
- AU-A- 1 145 699
- AU-A- 2 651 500
- AU-A- 5 509 999
- AU-A- 9 623 898
- AU-B- 686 891
- AU-B- 714 930
- HITOMI Y ET AL: "High efficiency prokaryotic expression and purification of a portion of the hepatitis C core protein and analysis of the immune response to recombinant protein in BALB/c mice." VIRAL IMMUNOLOGY, vol. 8, no. 2, 1995, pages 109-119, XP000978724 ISSN: 0882-8245
- LEE JAE WOO ET AL: "Identification of a domain containing B-cell epitopes in hepatitis C virus E2 glycoprotein by using mouse monoclonal antibodies." JOURNAL OF VIROLOGY, vol. 73, no. 1, January 1999 (1999-01), pages 11-18, XP002124766 ISSN: 0022-538X
- LAMONACA VINCENZO ET AL: "Conserved hepatitis C virus sequences are highly immunogenic for CD4+ T cells: Implications for vaccine development." HEPATOLOGY, vol. 30, no. 4, October 1999 (1999-10), pages 1088-1098, XP001027157 ISSN: 0270-9139
- KHEMKA VIMLA ET AL: "The capacity of a combined liposomal hepatitis B and C vaccine to stimulate humoral and cellular responses in mice." VIRAL IMMUNOLOGY, vol. 11, no. 2, 1998, pages 73-78, XP009006302 ISSN: 0882-8245
- POLAKOS N K ET AL: "Characterization of hepatitis C virus core-specific immune responses primed in rhesus macaques by a nonclassical ISCOM vaccine." JOURNAL OF IMMUNOLOGY (BALTIMORE, MD.: 1950) UNITED STATES 1 MAR 2001, vol. 166, no. 5, 1 March 2001 (2001-03-01), pages 3589-3598, XP001146112 ISSN: 0022-1767
- NAKANISHI T. ET AL.: 'Positively charged liposome functions as an efficient immunoadjuvant in inducing immune responses to soluble proteins' BIOCHEMICAL AND BIOPHYSICAL RESEARCH COMMUNICATIONS vol. 240, 1997, pages 793 - 797, XP002908766
- MOREIN B. AND BENGTSSON K.L.: 'Functional aspects of iscoms' IMMUNOLOGY AND CELL BIOLOGY vol. 76, 1998, pages 295 - 299, XP009006245
- SELBY M.J. ET AL.: 'Expression, identification and subcellular localization of the proteins encoded by the hepatitis C viral genome' JOURNAL OF GENERAL VIROLOGY vol. 74, 1993, pages 1103 - 1113, XP002918678

## Description

### FIELD OF THE INVENTION

The present invention relates generally to a vaccine composition and to an immunogenic complex for use in the vaccine composition. In particular, the invention relates to an immunogenic complex comprising a charged organic carrier, more particularly a negatively charged organic carrier, and a charged antigen, more particularly a positively charged antigen, wherein the charged antigen is a polyprotein, preferably the core protein, of Hepatitis C Virus (HCV) or a fragment thereof, or a fusion protein comprising said polyprotein or a fragment thereof. The vaccine compositions and immunogenic complexes of the present invention are useful, *inter alia*, as therapeutic and/or prophylactic agents for facilitating the induction of immune responses, and in particular a cytotoxic T-lymphocyte response, in the treatment of a disease condition which results from an HCV infection.

### BACKGROUND OF THE INVENTION

Hepatitis C virus (HCV) is now recognised as the leading cause of chronic liver disease and an estimated 170 million people are currently infected with the virus (Armstrong, G. L., M. J. Alter, G. M. McQuillan, and H. S. Margolis. 2000. Hepatology 31:777, Cohen, J. 1999. Science 285:26). Despite these alarming numbers, very few therapies are available for treatment and those available are of low efficacy. Improved therapies are desperately needed but their development has been hampered by the lack of a small animal model for infection and disease, and inefficient systems for cultivating the virus. Several studies have suggested that T cell-mediated immune responses to HCV infection can affect the outcome of HCV infection and disease (Missale, G., R. Bertoni, V. Lamonaca, A. Valli, M. Massari, C. Mori, M. G. Rumi, M. Houghton, F. Fiaccadori, and C. Ferrari. 1996. J. Clin. Invest. 98:706., Cooper, S. L., A. L. Erickson, E. J. Adams, J. Kansopon, A. J. Weiner, D. Y. Chien, M. Houghton, P. Parham, and C. M. Walker. 1999. Immunity 10:439, Lechner, F., D. K. Wong, P. R. Dunbar, R. Chapman, R. T. Chung, P. Dohrenwend, G. Robbins, R. Phillips, P. Klenerman, and B. D. Walker. 2000. J. Exp. Med. 191:1499). Furthermore there is an inverse correlation between the frequency of HCV specific cytotoxic T cells (CTLs) and viral load and the presence of HCV Core specific CTLs prior to interferon treatment has been associated with subsequent response of patients to this therapy (Nelson, D., C. Marousis, G. Davis, C. Rice, J. Wong, M. Houghton, and J. Lau. 1997. J. Immunol. 158:1473. Nelson, D. R., C. G. Marousis, T. Ohno, G. L. Davis, and J. Y. Lau. 1998. Hepatology 28:225). Thus, a vaccine capable of eliciting CTLs may be useful in control of HCV particularly as an adjunct to current therapies. The use of HCV proteins for vaccine development has been previously described (Houghton, EP Patent No. 0318216).

The adjuvant properties of saponin have been long known, as has its ability to increase antibody titres to immunogens. As used herein, the term "saponin" refers to a group of surface-active glycosides of plant origin composed of a hydrophilic region (usually several sugar chains) in association with a hydrophobic region of either steroid or triterpenoid structure. Although saponin is available from a number of diverse sources, saponins with useful adjuvant activity have been derived from the South American tree *Quillaja saponaria* (Molina). Saponin from this source was used to isolate a "homogeneous" fraction denoted "Quil A" (Dalsgaard, K., (1974), Arch. Gesamte Virusforsch. 44:243).

Dose-site reactivity is a major concern for both the veterinary and human use of Quil A in vaccine preparations. One way to avoid this toxicity of Quil A is the use of an immunostimulating complex (known as an ISCOM^{™}, an abbreviation for /mmuno Stimulating *COM*plex). This is primarily because Quil A is less reactive when incorporated into immunostimulating complexes, because its association with cholesterol in the complex reduces its ability to bind to cholesterol in cell membranes and hence its cell lytic effects. In addition, a lesser amount of Quil A is required to generate a similar level of adjuvant effect.

The immunomodulatory properties of the Quil A saponins and the additional benefits to be derived from these saponins when they are incorporated into an immunostimulating complex have been described in various publications, e.g. Cox and Cox, J.C. and Coulter, A.R. Advances in Adjuvant Technology and Application in Animal Parasite Control Utilising Biotechnology, Chapter 4, Editor Yong, W.K. CRC Press (1992); Cox, J.C. and Coulter, A.R. (1997) Vaccine, 15(3):248-256; Cox, J.C. and Coulter, A.R. (1999) BioDrugs 12(6):439-453); Dalsgaard, (1974) (supra); Morein et al., (1989) "Immunostimulating complex (ISCOM)", In "Vaccines: Recent Trends and Progress". G. Gregoriadis, A.C. Allison and G. Poster (Eds). Plenium Press, New York, p.153; Australian Patent Specifications Nos. 558258, 589915, 590904 and 632067.

Classic ISCOMs are formed by combination of cholesterol, saponin, phospholipid, and immunogens, such as viral envelope proteins. ISCOM matrix compositions (known as ISCOMATRIX^{™}) are formed identically, but without viral proteins. ISCOMs appear to stimulate both humoral and cellular immune responses. ISCOMs have been made with proteins from various viruses, including HSV-1, CMV, EBV, hepatitis B virus (HBV), rabies virus, and influenza virus, see for example, I.G. Barr et al., Adv. Drug Delivery Reviews, 32:247-271 (1998). It has been observed that where naked DNA or polypeptides from infectious agents are poorly immunogenic when given by themselves, inclusion within ISCOMs has increased their immunogenicity. Various proteins formulated with ISCOMs have been shown to induce CTL, mainly in rodent models. Berzofsky, (1991), Biotechnol. Ther. 2:123-135; Hsu et al., (1996), Vaccine 14:1159-1166; Lipford et al., (1994), Vaccine 12:73-80; Mowat et al., (1991), Immunology 72:317-322; Osterhaus et al., (1998), Dev. Biol. Stand. 92:49-58; Rimmelzwaan et al., (1997), J. Gen. Virol. 78 (pt.4):757-765; Sambhara et al., (1998), J. Infect. Dis. 177:1266-1274; Sambhara et al., (1997), Mech. Aging Dev. 96:157-169; Sjolander et al., (1997), Vaccine 15:1030-1038; Sjolander et al., (1998), J. Leukoc. Biol.. 64:713-723; Takahashi et al., (1990), Nature 344:873-875; Tarpey et al., (1996), Vaccine 14:230-236; Trudel et al., (1987), Vaccine 10:107-112; Verschoor et al., (1999), J. Virol. 73:3292-3300; Villacres-Eriksson, (1995), Clin. Exp. Immunol. 102:46-52; Zugel et al., (1995), Eur. J. Immunoll. 25:451-458.

Association between antigen and adjuvant is thought to be important for optimal induction of immune responses in particular CTL (Cox, J.C. and Coulter, A.R. (1999) BioDrugs, 12(6):439-445). A number of studies have been done which confirm this hypothesis including work with virosomes and ISCOMs^{™} (Ennis, F. A., Crux, J., Jameson, J., Klein, M., Burt, D. and Thipphawong, J. 1999. Virology 259: 256-261., Zurbriggen, R., Novak-Hofer, I., Seelig, A. and Gluck, R. (2000), Progress in lipid Research 39: 3-18. Typically association between ISCOM^{™} and antigen has been achieved by incorporation of amphipathic antigens into the ISCOM^{™} structure during formation (Morein, B., B. Sundquist, S. Hoglund, K. Dalsgaard, and A. Osterhaus. 1984. Nature 308:457). Incorporation was by hydrophobic interactions. However many antigens, such as the HCV core protein, were unable to be incorporated into ISCOMs^{™} by the classical method. More recently methods to associate antigens with a preformed protein-free immunostimulating complex (ISCOMATRIX^{™}) utilising chelating and electrostatic interactions have been developed (International Patent Applications Nos. PCT/AU98/00080 - WO 98/36772, and PCT/AU00/00110).

The Core protein of HCV, as well as the E1 and E2 envelope proteins, have been shown to be useful in immunizing against HCV (see, e.g., copending U.S. Patent Application Serial No. 08/823,980). The sequences for the envelope proteins also contain certain conserved regions, even in the hypervariable domains thereof, which provides increased utility for immunization against the various escape mutants responsible for chronic infections. There remains a need, however, for methods and compositions which increase the ability of these proteins and polypeptides to be immunogenic and to stimulate the development of broad and durable CTL active against HCV infection. in work leading up to the present invention, the inventors have developed an immunogenic complex based on the electrostatic association of an antigen of HCV and an organic carrier, such as an adjuvant. This electrostatic association permits co-delivery of the antigen and the organic carrier to the immune system, for the purpose of inducing an immune response, particularly a cytotoxic T-lymphocyte response, to the antigen.

### SUMMARY OF THE INVENTION

Throughout this specification and the claims which follow, unless the context requires otherwise, the word "comprise", and variations such as "comprises" and "comprising" will be understood to imply the inclusion of a stated integer or step or group of integers or steps but not the exclusion of any other integer or step of group of integers or steps.

One aspect of the present invention relates to an immunogenic complex comprising a negatively charged organic complex and a charged antigen, which organic complex and antigen are electrostatically associated, wherein the organic complex is a negatively charged adjuvant and comprises a saponin and cholesterol and wherein the charged antigen comprises (i) one or more immunogenic polypeptides from a region of Hepatitis C Virus (HCV) selected from Core, NS3, NS4a, NS4b, NS5a and NS5b, or (ii) a fusion protein comprising an immunogenic polypeptide from the Core region of HCV and a second immunogenic polypeptide selected from the E1, E2, NS3, NS4a, NS4b, NS5a and NS5b regions of HCV.

Preferably, the polyprotein is the core protein of HCV.

A further aspect of the present invention relates to a vaccine composition comprising as the active component an immunogenic complex according to the invention together with one or more pharmaceutically acceptable carriers and/or diluents.

Another further aspect of the present invention relates to the use of an immunogenic complex or vaccine composition according to the invention in the manufacture of a medicament for use in a method of treating or preventing an HCV infection in a mammal by eliciting, inducing or otherwise facilitating, an immune response to an HCV antigen.

Still a further aspect of the present invention relates to the use of an immunogenic complex or vaccine composition as hereinbefore described in the manufacture of a medicament for inhibiting, halting, delaying or preventing the onset or progression of an HCV infection.

Still yet another further aspect of the present invention relates to an immunogenic complex or vaccine composition according to the invention for use in a method of treating the human or animal body by therapy.

As described further below, the immunogenic complexes of the present invention may include, as the charged antigen, an HCV protein such as an HCV Core nucleocapsid protein, a nonstructural protein, immunogenic fragments of any of such proteins, or combinations of such proteins. Such fragments generally include polypeptides comprising epitopes recognizable by T cells. Preferred fragments comprise those fragments which are immunogenic when provided by themselves, or when included in an immunogenic complex of the present invention. As used throughout the specification, the term "polyprotein of HCV" or "HCV protein" is intended to include the full length protein as well as polypeptide fragments. An HCV protein such as an HCV Core nucleocapsid protein, a nonstructural protein, the E1 envelope protein, the E2 envelope protein, immunogenic fragments of any such proteins or combinations of such proteins may also be present in the immunogenic complexes of the present invention as fusion proteins, depending on which method of expression of the HCV protein is chosen. The sequences for these polypeptides and proteins are known (see, e.g. U.S. Patent No. 5,350,671). The invention also provides polypeptides that are homologous (i.e., have sequence identity) to these fragments. Depending on the particular fragment, the degree of sequence identity is preferably greater than 50% (e.g., 60%, 70%, 80%, 90%, 95%, 99% or more). These homologous polypeptides include mutants and allelic variants of the fragments.

### BRIEF DESCRIPTION OF THE DRAWINGS

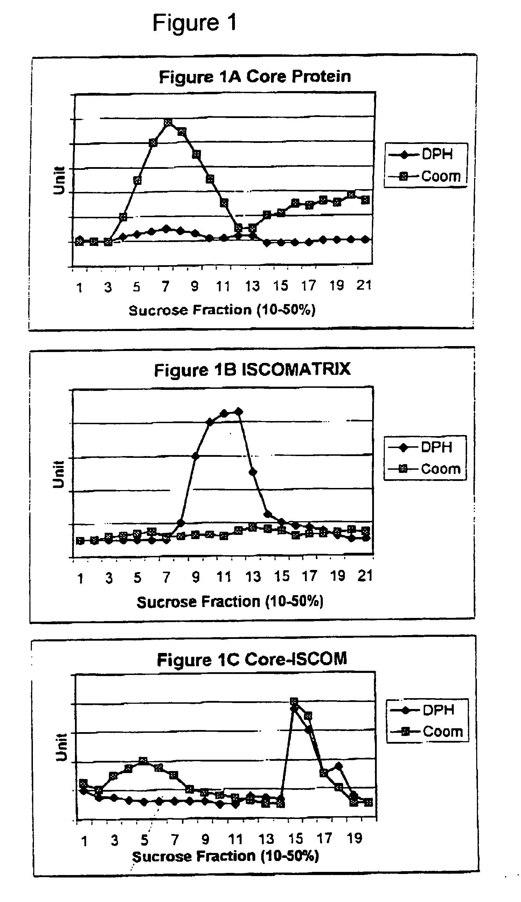

**Figure 1** is a graphical representation of the sucrose gradient analysis of Core-ISCOM^{™} formulations for core protein (Figure 1A), ISCOMATRIX^{™} (Figure 1B), and Core-ISCOM™ (Figure 1C).

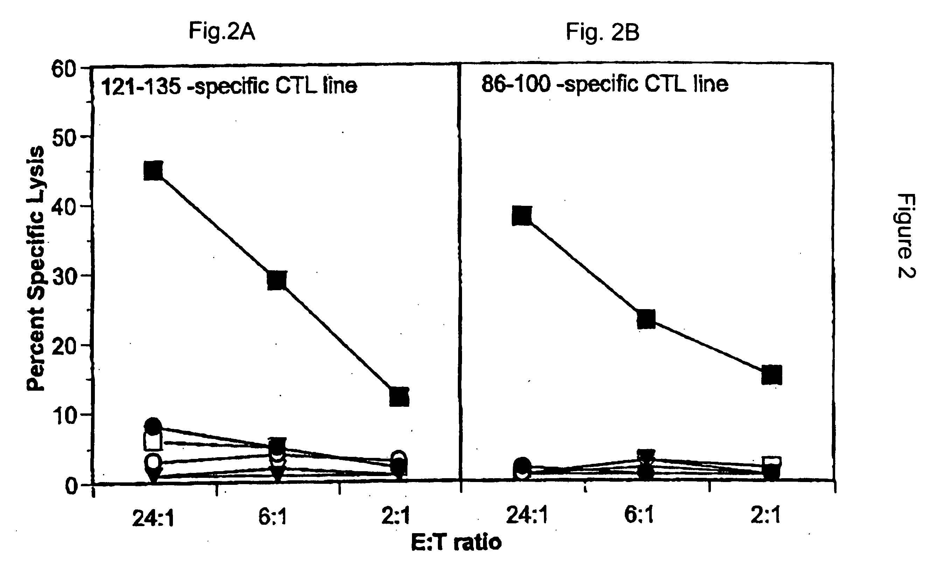

**Figure 2****:** B-LCLs from the two non-responders, HCV Core-ISCOM-immunized animals cannot present Core-derived peptides 121-135 and 86-100. Figure 2A: The 121-135-specific CTL line, established from animal DV037 was tested in a standard ⁵¹Cr release assay for its ability to lyse DV037 B-LCL target cells sensitized with peptide 121-135 (solid squares) or an irrelevant peptide (open squares), AY921 B-LCL target cells sensitized with peptide 121-135 (solid squares) or an irrelevant peptide (open circles), and BB231 B-LCL target cells sensitized with peptide 121-135 (solid triangles) or an irrelevant peptide (open triangles). Figure 2B: The 86-100 -specific CTL line was tested in a standard ⁵¹Cr release assay for its ability to lyse DV036 B-LCL target cells sensitized with peptide 86-100 (solid squares) or an irrelevant peptide (open squares), AY921 B-LCL target cells sensitized with peptide 86-100 (solid circles) or an irrelevant peptide (open circles), and BB231 B-LCL target cells sensitized with peptide 86-100 (solid triangles) or an irrelevant peptide (open triangles).

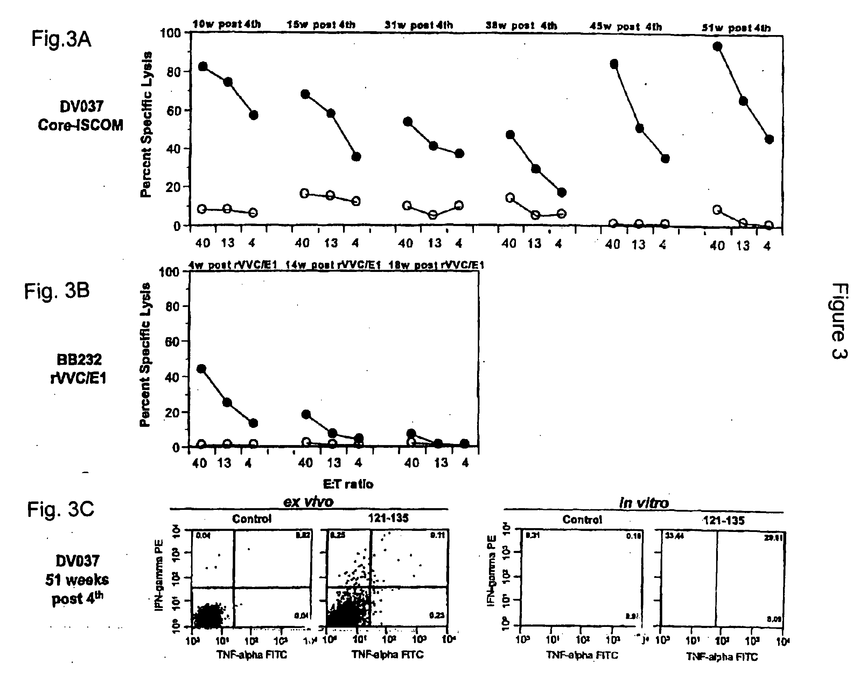

**Figure 3** shows the longevity of the CTL responses primed by vaccination. PBMCs from DV037 (Figure 3A) and BB232 (Figure 3B) were restimulated *in vitro* with the epitopic peptide 121-135. After CD8+ enrichment, cells were tested for cytotoxic activity against autologous B-LCLs sensitized with the epitopic peptide 121-135 (solid circles) or an irrelevant peptide (open circles). Figure 3C shows the results of an experiment where freshly isolated PBMCs from DV037, 51 weeks after its last immunization (two left panels), or *in vitro*-restimulated PBMCs from the same time point (two right panels) were restimulated for 12 hours with peptide 121-135 or a control peptide and stained for surface CD8 and intracellular IFN-γ and TNF-α. Lymphocytes were gated by side vs. forward scatter light and then for CD8-PerCP. Plots show log fluorescence intensity for TNF-α-FITC and IFN-γ-PE.

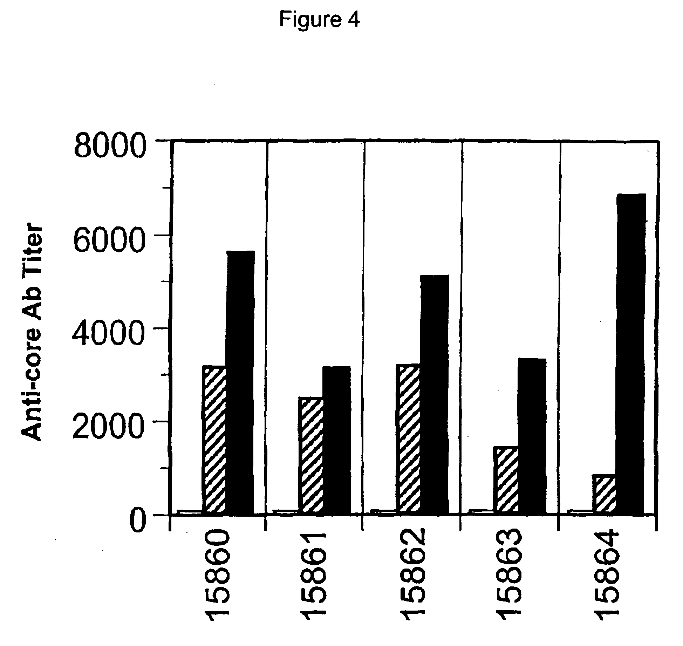

**Figure 4** shows antibody titers against Core in the serum of immunized animals. Open bars, pre-immunization; striped bars, 2 weeks post 2^{nd} immunization; filled bars, 2 weeks post 3^{rd} immunization.

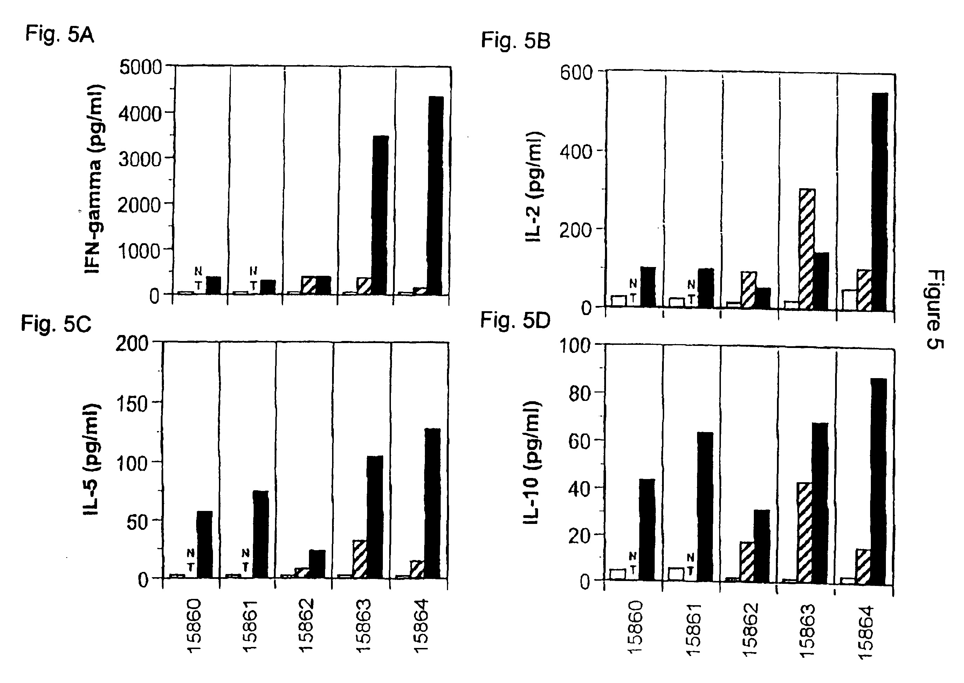

**Figure 5** shows Th-1 and Th2-type cytokines in Core-ISCOM-immunized animals. The level of IFN-γ (Figure 5A), IL-2 (Figure 5B), IL-5 (Figure 5C) and IL-10 (Figure 5D) was measured by specific ELISA in cell-free supernatant of freshly isolated PBMCs stimulated for 48h as described in the examples. Open bars, pre-immunization; striped bars, 2 weeks post 2^{nd} immunization; filled bars, 2 weeks post 3^{rd} immunization. NT: Not Tested.

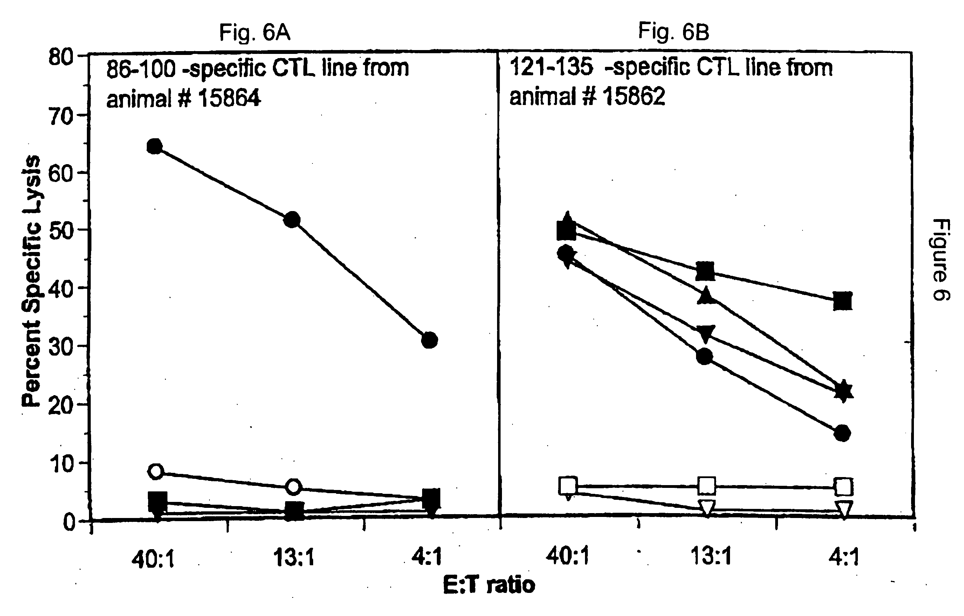

**Figure 6** shows MHC class I restriction of peptides 121-135 and 86-100 CTLs. (Figure 6A) shows the results of an experiment where peptide 86-100-specific CTL line derived from animal 15864 was tested in a standard ⁵¹Cr release assay for its ability to lyse peptide 86-100 -sensitized B-LCL target cells derived from animals DV036 (solid squares), 15864 (solid circles), 15860 (solid triangles) and 15861 (open circles). Figure 6B shows the results from an experiment where peptide 121-135-specific CTL line derived from animal 15862 was tested in a standard ⁵¹Cr release assay for its ability to lyse peptide 121-135-sensitized B-LCL target cells derived from animals DV037 (solid squares), BB232 (solid triangles),15862 (solid inverted triangles), 15863 (solid circles), 15861 (open squares) and 15860 (open inverted triangles).

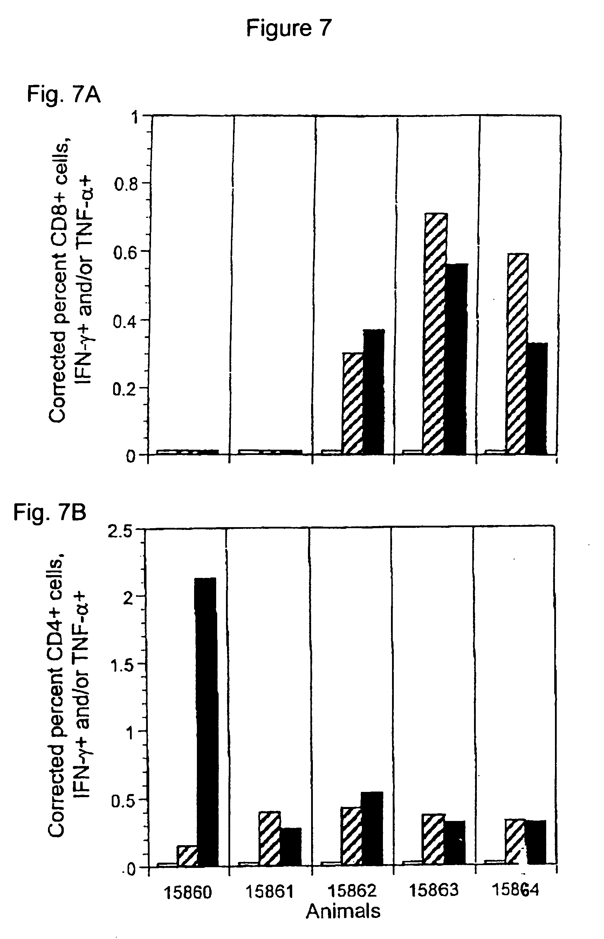

**Figure 7** shows a quantification of the CD8+ and CD4+ T cell responses in Core-ISCOM-immunized animals. Freshly isolated PBMCs were restimulated *ex vivo* with rVVC/E1 or VVwt-infected autologous B-LCLs (Figure 7A) or with the recombinant Core protein of an *E. coli* control (Figure 7B). Cells were then stained for surface CD8 or CD4, and intracellular IFN-γ and TNF-α as described in the examples. Lymphocytes were gated by side vs. forward scatter light and then for CD8-PerCP (Figure 7A) or CD4-APC (Figure 7B). In Figure 7A, the corrected percent of CD8+ T cells with detectable IFN-γ and/or TNF-α was calculated as (% CD8+ T cells restimulated with rVVC/E1 that were IFN-γ and/or TNF-α+) - (% CD8+ T cells restimulated with VVwt that were IFN-γ and/or TNF-α+). In Figure 7B, the corrected percent of CD4+ T cells with detectable IFN-λ and/or TNF-α was calculated as (% CD4+ T cells restimulated with Core that were IFN-γ and/or TNFα-+) - (% CD4+ T cells restimulated with the *E. coli* that were IFN-γ and/or TNF-α+). Open bars, pre-immunization; striped bars, 2 weeks post 2^{nd} immunization; filled bars, 2 weeks post 3^{rd} immunization.

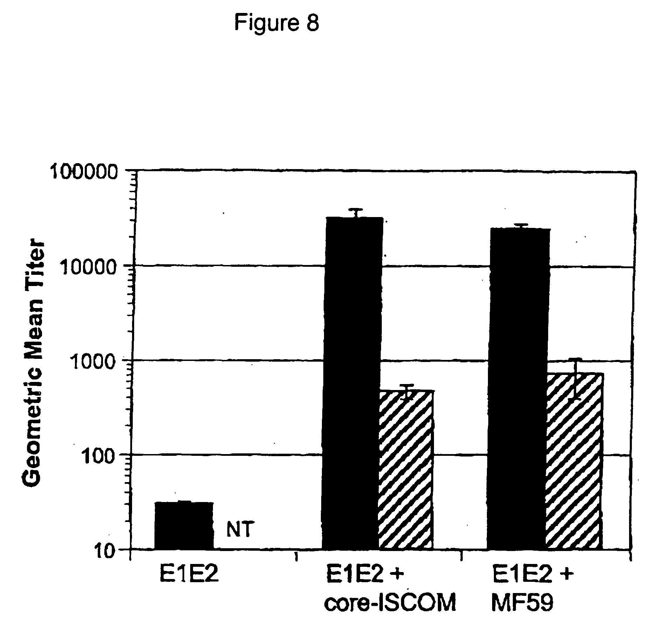

**Figure 8** shows that Core-ISCOM can serve as an adjuvant for E1E2. Mice (10 animals per group) were immunized with 2g of soluble E1 E2 alone, 2 g of soluble E1E2 + 2g of Core-ISCOM, or 2 g of soluble E1 E2 adjuvanted with MF59. Mice were bled two weeks post 3^{rd} immunization. Anti-E2 (filled bars) and anti-CD81 titers (striped bars) are presented as the geometric mean of the titers obtained from the individual mice from each group. NT: Not Tested.

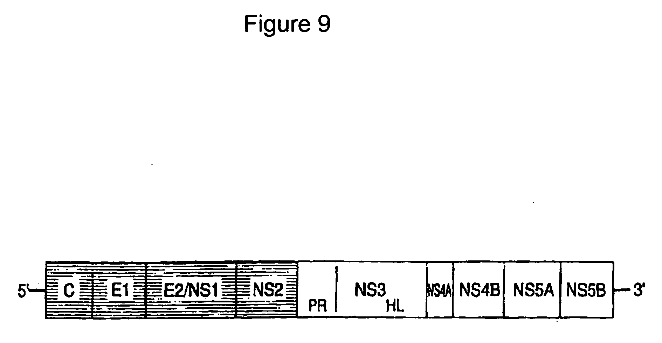

**Figure 9** is a diagrammatic representation of the HCV genome, depicting the various regions of the polyprotein from which the present proteins for use with the ISCOMs are derived.

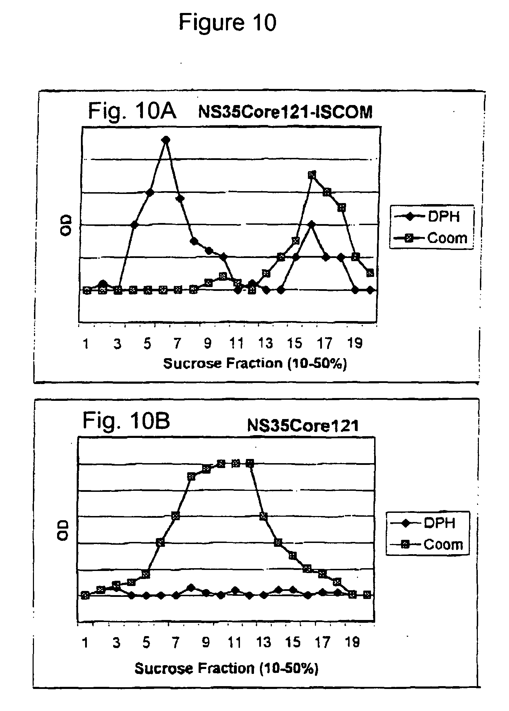

**Figure 10** is a graphical representation of the sucrose gradient analysis of NS35 Core 121-ISCOM™ formulations for NS35 Core 121-ISCOM™. (Figure 10A) and NS35 Core 121 protein (Figure 10B).

### DETAILED DESCRIPTION OF THE PREFERRED EMBODIMENTS

The present invention is predicated, in part, on the development of an immunogenic complex formulation which utilises electrostatic interactions to associate an antigen of HCV and a carrier thereby facilitating, *inter alia*, the co-delivery of these molecules to the immune system. The immunogenic complexes of the present invention are particularly suitable for use in facilitating the stimulation of cytotoxic T-lymphocyte responses.

Accordingly, one aspect of the present invention relates to an immunogenic complex comprising a charged organic carrier and a charged antigen, which organic carrier and antigen are electrostatically associated, and wherein the charged antigen is a polyprotein of Hepatitis C Virus (HCV), preferably the core protein of HCV, or a fragment thereof, or a fusion protein comprising said polyprotein or a fragment thereof.

Reference to a "complex" should be understood as describing an entity of two or more different interacting chemical components.

Reference to a "charged" organic carrier or antigen should be understood as a reference to an organic carrier or antigen which exhibits an overall positive electrical charge or an overall negative electrical charge. By "overall" is meant the summation of the individual positive and negative charges which a given molecule comprises. Where the summation of the individual positive and negative charges results in overall electrical neutrality, the molecule is not regarded as "charged" within the context of the present invention. Preferably, the organic carrier comprises an overall negative charge.

Accordingly, the present invention more particularly provides an immunogenic complex as described above, wherein the charged organic carrier is a negatively charged organic carrier.

Reference to "electrostatically associated" is a reference to the organic carrier and the antigen being linked, bound or otherwise associated by means which include electrostatic interaction. Accordingly, it should be understood that in some instances the electrostatic interaction will be the only attractive force which results in complexing of the antigen and the organic carrier. However, in other instances the formation of the electrostatic interaction may also lead to, or be associated with, the formation of other interactive forces.

The terms "polyprotein", "protein" and "polypeptide" are used interchangeably herein and refer to a polymer of amino acid residues and are not limited to a minimum length of the product. Thus, peptides, oligopeptides, dimers, multimers, and the like, are included within the definition. Both full-length proteins and fragments thereof are encompassed by the definition. The terms also include postexpression modifications of the polypeptide, for example, glycosylation, acetylation, phosphorylation and the like. Furthermore, for purposes of the present invention, a "polypeptide" refers to a protein which includes modifications, such as deletions, additions and substitutions (generally conservative in nature), to the native sequence, so long as the protein maintains the desired activity. These modifications may be deliberate, as through site-directed mutagenesis, or may be accidental, such as through mutations of hosts which produce the proteins or errors due to PCR amplification.

In particular, as shown in Figure 9, several proteins are encoded by the HCV genome. The order and nomenclature of the cleavage products of the HCV polyprotein is as follows: NH₂-C-E1-E2-NS2-NS3-NS4a-NS4b-NS5a-NS5b-COOH. Initial cleavage of the polyprotein is catalyzed by host proteases which liberate three structural proteins, the N-terminal nucleocapsid protein (termed "Core") and two envelope glycoproteins, "E1" (also known as E) and "E2" (also known as E2/NS1), as well as nonstructural (NS) proteins that contain the viral enzymes. The NS regions are termed NS2, NS3, NS4 and NS5. NS2 is an integral membrane protein with proteolytic activity. NS2, either alone or in combination with NS3, cleaves the NS2-NS3 sissle bond which in turn generates the NS3 N-terminus and releases a large polyprotein that includes both serine protease and RNA helicase activities. The NS3 protease serves to process the remaining polyprotein. Completion of polyprotein maturation is initiated by autocatalytic cleavage at the NS3-NS4a junction, catalyzed by the NS3 serine protease. Subsequent NS3-mediated cleavages of the HCV polyprotein appear to involve recognition of polyprotein cleavage junctions by an NS3 molecule of another polypeptide. In these reactions, NS3 liberates an NS3 cofactor (NS4a), two proteins with unknown function (NS4b and NS5a), and an RNA-dependent RNA polymerase (NS5b).

As explained above, any of a number of HCV polypeptides derived from the HCV polyprotein may be used in the immunogenic complexes of the present invention. Thus, these complexes may contain polypeptides derived from the HCV Core nucleocapsid protein, a nonstructural protein, the E1 envelope protein, the E2 envelope protein, polypeptide fragments of any of such proteins, or combinations of such proteins. Such fragments may be polypeptides comprising epitopes recognizable by T cells. Preferred fragments comprise those fragments which are immunogenic when provided by themselves, or when included in the immunogenic complex of the present invention.

Preferably, the immunogenic complex of the present invention comprises the core protein of HCV, or an immunogenic fragment thereof. The core protein of HCV has a pl of 10, making it highly positively charged at neutral and acidic pH. Reference to the "core protein" of HCV should be understood as including a reference to derivatives and equivalents of the core protein.

The polypeptide for use in the immunogenic complex of the present invention need not be physically derived from HCV, but may be synthetically or recombinantly produced using conventional techniques of molecular biology, microbiology, recombinant DNA, and immunology, which are within the skill of the art. Such techniques are explained fully in the literature, e.g. Sambrook Molecular Cloning : A Laboratory Manual, Second Edition (1989); DNA Cloning, Volumes I and II (D.N. Glovere, ed., 1985); Oligonucleotide Synthesis (M.J. Gait, ed., 1986); Nucleic Acid Hybridization (B.D. Hames & S.J. Higgins, eds., 1984); Transcription and Translation (B.D. Hames & S.J. Higgins, eds., 1984); Animal Cell Culture (R.I. Freshney, ed., 1986); Immobilized Cells and Enzymes (IRL Press, 1986); B. Perbal, A Practical Guide to Molecular Cloning (1984); the Methods in Enzymology series (Academic Press, Inc.), especially volumes 154 and 155; Gene Transfer Vectors for Mammalian Cells (J.H. Miller and M.P. Calos, eds., 1987, Cold Spring Harbor Laboratory); Mayer and Walker, eds., (1987), Immunochemical Methods in Cell and Molecular Biology (Academic Press, London); Scopes, (1987), Protein Purification: Principles and Practice, Second Edition (Springer-Verlag, N.Y.), and Handbook of Experimental Immunology, Volumes I-IV (D.M. Weir and C.C. Blackwell, eds., 1986).

Moreover, the polypeptide may be derived from any of the various known HCV strains, such as from strains 1, 2, 3 or 4 of HCV. A number of conserved and variable regions are known between these strains and, in general, the amino acid sequences of epitopes derived from these regions will have a high degree of sequence homology, e.g., amino acid sequence homology of more than 30%, preferably more than 40%, when the two sequences are aligned. Thus, for example, the term "Core" polypeptide refers to the native Core protein from any of the various HCV strains, as well as Core analogs, muteins and immunogenic fragments, as defined further below.

Reference to "derivative and equivalents" should be understood as a reference to chemical equivalents, mutants, homologs and analogs from natural, synthetic or recombinant sources. Derivatives may be derived from insertion, deletion or substitution of amino acids. Amino acid insertional derivatives include amino and/or carboxylic terminal fusions as well as intrasequence insertions of single or multiple amino acids. Insertional amino acid sequence variants are those in which one or more amino acid residues are introduced into a predetermined site in the protein although random insertion is also possible with suitable screening of the resulting product. Deletional variants are characterised by the removal of one or more amino acids from the sequence. Substitutional amino acid variants are those in which one residue in the sequence has been removed and a different residue inserted in its place. "Equivalents" can act as a functional analog of the subject antigen. Chemical equivalents may not necessarily be derived from the subject antigen but may share certain conformational similarities. Alternatively, chemical equivalents may be designed to mimic certain physiochemical properties of the subject antigen. Equivalents may be chemically synthesised or may be detected following, for example, natural product screening. Homologs contemplated herein include, but are not limited to, molecules derived from different species.

The present invention also extends to an immunogenic complex as described above wherein the charged antigen is a fragment of an HCV protein. By "fragment" is intended a polypeptide consisting of only a part of the intact full-length protein sequence and structure. The fragment can include a C-terminal deletion and/or an N-terminal deletion of the native polypeptide. An "immunogenic fragment" of a particular HCV protein will generally include at least about 5-10 contiguous amino acid residues of the full-length molecule, preferably at least about 15-25 contiguous amino acid residues of the full-length molecule, and most preferably at least about 20-50 or more contiguous amino acid residues of the full-length molecule, that define an epitope, or any integer between 5 amino acids and the full-length sequence, provided that the fragment in question retains the ability to elicit an immune response as defined below. For example, preferred immunogenic fragments, include but are not limited to fragments of the core protein of HCV that comprise, e.g., amino acids 10-45, 10-53, 67-88, 81-130, 86-100, 120-130, 121-135 and 121-170 of the polyprotein, numbered relative to the HCV-1a sequence presented in Choo et al. (1991) Proc Natl Acad Sci USA 88:2451, as well as defined epitopes derived from the c33c region of the HCV polyprotein, as well as any of the other various epitopes identified from the HCV core, E1, E2, NS3 and NS4 regions. See, e.g., Chien et al. Proc. Natl. Acad. Sci. USA (1992) 89:10011-10015; Chien et al. J. Gastroent. Hepatol. (1993) 8:S33-39; Chien et al. International Publ. No. WO 93/00365; Chien, D.Y. International Publ. No. WO 94/01778; allowed U.S. Patent Application Serial Nos. 08/403,590 and 08/444,818.

The term "epitope" as used herein refers to a sequence of at least about 3 to 5, preferably about 5 to 10 or 15, and not more than about 1,000 amino acids (or any integer therebetween), which define a sequence that by itself or as part of a larger sequence, will stimulate a host's immune system to make a cellular antigen-specific immune response when the antigen is presented, or a humoral antibody response. An epitope for use in the subject invention is not limited to a polypeptide having the exact sequence of the portion of the parent protein from which it is derived. Indeed, viral genomes are in a state of constant flux and contain several variable domains which exhibit relatively high degrees of variability between isolates. Thus the term "epitope" encompasses sequences identical to the native sequence, as well as modifications to the native sequence, such as deletions, additions and substitutions (generally conservative in nature).

Regions of a given polypeptide that include an epitope can be identified using any number of epitope mapping techniques, well known in the art. See, e.g., Epitope Mapping Protocols in Methods in Molecular Biology, Vol. 66 (Glenn E. Morris, Ed., 1996) Humana Press, Totowa, New Jersey. For example, linear epitopes may be determined by e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Patent No. 4,708,871; Geysen et al. (1984) Proc. Natl. Acad. Sci. USA 81:3998-4002; Geysen et al. (1986) Molec. Immunol. 23:709-715, all incorporated herein by reference in their entireties. Similarly, conformational epitopes are readily identified by determining spatial conformation of amino acids such as by, e.g., x-ray crystallography and 2-dimensional nuclear magnetic resonance. See, e.g., *Epitope Mapping Protocols, supra*. Antigenic regions of proteins can also be identified using standard antigenicity and hydropathy plots, such as those calculated using, e.g., the Omiga version 1.0 software program available from the Oxford Molecular Group. This computer program employs the Hopp/Woods method, Hopp et al., Proc. Natl. Acad. Sci USA (1981) 78:3824-3828 for determining antigenicity profiles, and the Kyte-Doolittle technique, Kyte et al., J. Mol. Biol. (1982) 157:105-132 for hydropathy plots.

As used herein, the term "conformational epitope" refers to a portion of a full-length protein, or an analog or mutein thereof, having structural features native to the amino acid sequence encoding the epitope within the full-length natural protein. Native structural features include, but are not limited to, glycosylation and three dimensional structure. Preferably, a conformational epitope is produced recombinantly and is expressed in a cell from which it is extractable under conditions which preserve its desired structural features, e.g. without denaturation of the epitope. Such cells include bacteria, yeast, insect, and mammalian cells. Expression and isolation of recombinant conformational epitopes from the HCV polyprotein are described in e.g., International Publication Nos. WO 96/04301, WO 94/01778, WO 95/33053, WO 92/08734, which applications are herein incorporated by reference in their entirety.

As used herein the term "T-cell epitope" refers to a feature of a peptide structure which is capable of inducing T-cell immunity towards the peptide structure or an associated hapten. T-cell epitopes generally comprise linear peptide determinants that assume extended conformations within the peptide-binding cleft of MHC molecules, (Unanue et al., Science (1987) 236:551-557). Conversion of polypeptides to MHC class II-associated linear peptide determinants (generally between 5 - 14 amino acids in length) is termed "antigen processing" which is carried out by antigen presenting cells (APCs). More particularly, a T-cell epitope is defined by local features of a short peptide structure, such as primary amino acid sequence properties involving charge and hydrophobicity, and certain types of secondary structure, such as helicity, that do not depend on the folding of the entire polypeptide. Further, it is believed that short peptides capable of recognition by helper T-cells are generally amphipathic structures comprising a hydrophobic side (for interaction with the MHC molecule) and a hydrophilic side (for interacting with the T-cell receptor), (Margalit et al., Computer Prediction of T-cell Epitopes, New Generation Vaccines Marcel-Dekker, Inc, ed. G.C. Woodrow et al., (1990) pp. 109-116) and further that the amphipathic structures have an α-helical configuration (see, e.g., Spouge et al. J. Immunol. (1987) 138:204-212; Berkower et al. J. Immunol. (1986) 136:2498-2503).

Hence, segments of proteins which include T-cell epitopes can be readily predicted using numerous computer programs. (See e.g., Margalit et al., Computer Prediction of T-cell Epitopes, New Generation Vaccines Marcel-Dekker, Inc, ed. G.C. Woodrow et al., (1990) pp. 109-116). Such programs generally compare the amino acid sequence of a peptide to sequences known to induce a T-cell response, and search for patterns of amino acids which are believed to be required for a T-cell epitope.

In general, "identity" refers to an exact nucleotide-to-nucleotide or amino acid-to-amino acid correspondence of two polynucleotides or polypeptide sequences, respectively. Percent identity can be determined by a direct comparison of the sequence information between two molecules by aligning the sequences, counting the exact number of matches between the two aligned sequences, dividing by the length of the shorter sequence, and multiplying the result by 100. Readily available computer programs can be used to aid in the analysis, such as ALIGN, Dayhoff, M.O. in Atlas of Protein Sequence and Structure M.O. Dayhoff ed., 5 Suppl. 3:353-358, National biomedical Research Foundation, Washington, DC, which adapts the local homology algorithm of Smith and Waterman Advances in Appl. Math. 2:482-489, 1981 for peptide analysis. Programs for determining nucleotide sequence identity are available in the Wisconsin Sequence Analysis Package, Version 8 (available from Genetics Computer Group, Madison, WI) for example, the BESTFIT, FASTA and GAP programs, which also rely on the Smith and Waterman algorithm. These programs are readily utilized with the default parameters recommended by the manufacturer and described in the Wisconsin Sequence Analysis Package referred to above. For example, percent identity of a particular nucleotide sequence to a reference sequence can be determined using the homology algorithm of Smith and Waterman with a default scoring table and a gap penalty of six nucleotide positions.

Another method of establishing percent identity in the context of the present invention is to use the MPSRCH package of programs copyrighted by the University of Edinburgh, developed by John F. Collins and Shane S. Sturrok, and distributed by IntelliGenetics, Inc. (Mountain View, CA). From this suite of packages the Smith-Waterman algorithm can be employed where default parameters are used for the scoring table (for example, gap open penalty of 12, gap extension penalty of one, and a gap of six). From the data generated the "Match" value reflects "sequence identity." Other suitable programs for calculating the percent identity or similarity between sequences are generally known in the art, for example, another alignment program is BLAST, used with default parameters. For example, BLASTN and BLASTP can be used using the following default parameters: genetic code = standard; filter = none; strand = both; cutoff = 60; expect = 10; Matrix = BLOSUM62; Descriptions = 50 sequences; sort by = HIGH SCORE; Databases = non-redundant, GenBank + EMBL + DDBJ + PDB + GenBank CDS translations + Swiss protein + Spupdate + PIR. Details of these programs can be found at the following internet address: http://www.ncbi.nlm.gov/cgi-binBLAST.

The sequences for these polypeptides and proteins are known (see, e.g., U.S. Patent No. 5,350,671). For example, a number of general and specific immunogenic polypeptides, derived from the HCV polyprotein, have been described. See, e.g., Houghton et al., European Publ. Nos. 318,216 and 388,232; Choo et al. Science (1989) 244:359-362; Kuo et al. Science (1989) 244:362-364; Houghton et al. Hepatology (1991) 14:381-388; Chien et al. Proc. Natl. Acad. Sci. USA (1992) 89:10011-10015; Chien et al. J. Gastroent. Hepatol. (1993) 8:S33-39; Chien et al., International Publ. No. WO 93/00365; Chien, D.Y., International Publ. No. WO 94/01778. These publications provide an extensive background on HCV generally, as well as on the manufacture and uses of HCV polypeptide immunological reagents.

It should be noted that for convenience, the various HCV regions are generally defined with respect to the amino acid number relative to the polyprotein encoded by the genome of HCV-1a, as described in Choo et al. (1991) Proc Natl Acad Sci USA 88:2451, with the initiator methionine being designated position 1. However, the polypeptides for use with the present invention are not limited to those derived from the HCV-1a sequence. In this regard, the corresponding regions in another HCV isolate can be readily determined by aligning sequences from the two isolates in a manner that brings the sequences into maximum alignment.

For example, HCV polypeptides derived from the Core region, such as polypeptides derived from the region found between amino acids 1-191; amino acids 10-53; amino acids 10-45; amino acids 67-88; amino acids 86-100; 81-130; amino acids 121-135; amino acids 120-130; amino acids 121-170; and any of the Core epitopes identified in, e.g., Houghton et al., U.S. Patent No. 5,350,671; Chien et al. Proc. Natl. Acad. Sci. USA (1992) 89:10011-10015; Chien et al. J. Gastroent. Hepatol. (1993) 8:S33-39; Chien et al., International Publ. No. WO 93/00365; Chien, D.Y., international Publ. No. WO 94/01778; and allowed U.S. Patent Application Serial Nos. 08/403,590 and 08/444,818 will find use in the immunogenic complexes of the present invention.

HCV polypeptides derived from the envelope of HCV, including polypeptides derived from the E1 and E2 regions, as well as fusions between E1 and E2 will also find use herein. Particularly, the HCV envelope glycoproteins E1 and E2 form a stable complex that is co-immunoprecipitable (Grakoui et al. (1993) J. Virol. 67:1385-1395; Lanford et al. (1993) Virology 197:225-235; Ralston et al. (1993) J. Virol. 67:6753-6761). The HCV E1 and E2 glycoproteins have been shown to be protective in primate studies. (Choo et al. (1994) Proc. Natl. Acad. Sci. USA 91:1294-1298). The mature E1 region of HCV1 begins at approximately amino acid 192 of the polyprotein and continues to approximately amino acid 383. The mature E2 region of HCV1 begins at approximately amino acid 384-385 and extends as far as approximately amino acid residue 746 (see, Lin et al. J. virol. (1994) 68:5063-5073).

Additionally, polypeptides derived from the nonstructural regions of the virus will also find use herein. The NS3/4a region of the HCV polyprotein has been described and the amino acid sequence and overall structure of the protein are disclosed in Yao et al. Structure (November 1999) 7:1353-1363. See, also, Dasmahapatra et al., U.S. Patent No. 5,843,752. As explained above, either the native sequence or immunogenic analogs can be used in the subject formulations. Dasmahapatra et al., U.S. Patent No. 5,843,752 and Zhang et al., U.S. Patent No. 5,990,276, both describe analogs of NS3/4a and methods of making the same.

Additionally, multiple epitope fusion antigens (termed "MEFAs"), as described in International Publ. No. WO 97144469, may be associated with the immunogenic complexes. Such MEFAs include multiple epitopes derived from two or more of the various viral regions. The epitopes are preferably from more than one HCV strain, thus providing the added ability to protect against multiple strains of HCV in a single vaccine.

Moreover, polypeptides for use in the immunogenic complexes of this invention may be derived from the NS3 region of the HCV polyprotein. A number of such polypeptides are known, including, but not limited to polypeptides derived from the c33c and c100 regions, as well as fusion proteins comprising an NS3 epitope, such as c25. These and other NS3 polypeptides are useful in the present compositions and are known in the art and described in, e.g., Houghton et al, U.S. Patent No. 5,350,671; Chien et al. Proc. Natl. Acad. Sci. USA (1992) 89:10011-10015; Chien et al. J. Gastroent. Hepatol. (1993) 8:S33-39; Chien et al., International Publ. No. WO 93/00365; Chien, D.Y., International Publ. No. WO 94/01778; and allowed U.S. Patent Application Serial Nos. 08/403,590 and 08/444,818.

It is readily apparent that a multitude of HCV polypeptides may be used in the immunogenic complex formulations or may be coadministered therewith, in order to provide a cellular immune response against the HCV antigen in question.

The present invention further extends to an immunogenic complex as described above wherein the charged protein is a fusion protein comprising the core or another protein of HCV or a fragment thereof. Reference to a "fusion protein" should be understood as a reference to a fusion in which the HCV core or other protein or fragment is operatively linked to another peptide, polypeptide or protein. The term "operatively linked" is intended to indicate that the HCV core or other protein or fragment and the other peptide, polypeptide or protein are fused in-frame to each other, either directly or indirectly through a linker peptide or polypeptide, with the fusion being at either the N-terminal end or the C-terminal end of the HCV core or other protein or fragment.

The fusion protein may comprise a tag protein or peptide moiety such as a hexa-his (His)₆ moiety, glutathione-S-transferase (GST) moiety or a FLAG moiety.

Preferably, however, the fusion protein comprises a second immunogenically active peptide, polypeptide or protein which may be derived from HCV, or some other viral, bacterial, fungal or similar organism.

The linker peptide or polypeptide, where present in the fusion protein, may comprise a sequence of from 1 to 50, preferably 1 to 20, and more preferably 1 to 5 amino acid residues.

Reference throughout this specification to "organic carrier" should be understood as a reference to any molecule, aggregate or complex of molecules, compound or other entity which, when an antigen is associated with it, facilitates the induction of an immune response, and in particular a cytotoxic T-lymphocyte response, to the antigen. The subject carrier is "organic" and, in this regard, "organic" should be understood as a compound of carbon whether naturally, recombinantly or synthetically obtained or derived. In a particularly preferred embodiment the organic carrier is an adjuvant. By "adjuvant" is meant any molecule, aggregate or complex of molecules, compound or other entity which functions to stimulate, enhance or otherwise up-regulate any one or more aspects of the immune response. For example, the adjuvant may induce inflammation thereby attracting immune response cells to the site of antigen localisation. Alternatively, the adjuvant may slowly release the antigen thereby providing on-going stimulation of the immune system.

Examples of charged organic carriers which are adjuvants suitable for use in the present invention include, but are not limited to, saponin, saponin complexes, any one or more components of the immunostimulating complex of saponin, cholesterol and lipid known as ISCOMATRIX^{™} (for example the saponin component and/or the phospholipid component), liposomes or oil-in-water emulsions. [The composition and preparation of ISCOMATRIX^{™} is described in detail in International Patent Application Number PCT/SE86/00480, Australian Patent Numbers 558258 and 632067 and European Patent Publication No. 0 180 564]. Further examples of adjuvants include, but are not limited to, those detailed in the publications of Cox and Coulter, 1992, 1997 and 1999. It should be understood that the subject organic carrier may be naturally occurring or it may be synthetically or recombinantly derived.

Accordingly, the present invention still more preferably provides an immunogenic complex as described above, wherein the charged organic carrier is a negatively charged adjuvant.

Preferably, said adjuvant comprises saponin or a saponin complex. More preferably, said saponin complex is ISCOMATRIX^{™}.

The organic carrier of the present invention may also be, in its initial or natural form, negatively charged, positively charged or neutral. Increasing the degree of negative charge (for example, where the organic carrier is only weakly negatively charged) or converting a neutral or positively charged organic carrier to a negatively charged organic carrier may also be achieved by any suitable method known to those skilled in the art. For example, where the organic carrier is an oil-in-water emulsion, incorporation of any anionic surfactant with a non-polar tail will impart an overall negative charge to the emulsion due to insertion of the tail of the surfactant into the oil droplet which thereby leaves the negatively charged head group exposed. The negative charge of a saponin complex adjuvant may be increased, for example, by the addition of negatively charged lipid during complex formation.

Examples of detergents which can increase the negative charge of a carrier include, but are not limited to cholic acid, deoxycholic acid, taurocholic acid and taurodeoxycholic acid. Examples of lipids which can increase the negative charge of a carrier include, but are not limited to, phospholipids (preferably phosphatidyl inositol, phosphatidyl serine, phosphatidyl glycerol and phosphatidic acid and most preferably cardiolipin) and bacterial lipids (preferably monophosphoryl lipid A(MPL) and most preferably diphosphoryl lipid A, such as OM174 as described in International Patent Publication No. WO 95/14026).

Without limiting the present invention in any way, the inventors have determined that where the subject charged organic carrier and charged antigen are naturally negatively and positively charged, respectively, the object of the invention can be achieved. However, a still more effective immunogenic complex may be achieved if the subject naturally negatively charged organic carrier is rendered more negatively charged (preferably by addition of cardiolipin or diphosphoryl lipid A).

Accordingly, in one preferred embodiment there is provided an immunogenic complex as described above, wherein the negatively charged adjuvant is a naturally negatively charged adjuvant which has been modified to increase the degree of its negative charge.

Reference to an adjuvant being "naturally" negatively charged, should be understood as a reference to the charge which the molecule bears upon its creation - whether that be by natural, recombinant or synthetic means. Modification to increase the degree of charge can be achieved by any suitable technique as hereinbefore discussed. Preferably, the subject adjuvant is rendered more negative via the addition of cardiolipin or diphosphoryl lipid A.

The present invention is predicated, in part, on the formation of immunogenic complexes via the electrostatic association, preferably, of a negatively charged organic carrier with a positively charged antigen. The administration of such a complex to a subject facilitates the induction of a significantly better immune response than would be achieved were the adjuvant and antigen administered simultaneously but in a non-associated form. In particular, the administration of an antigen associated with an adjuvant, according to the present invention, facilitates the induction of a cytotoxic T-lymphocyte response to the antigen. However, humoral and other cellular responses can also be enhanced.

Generally, in the immunogenic complex of the present invention, the ratio of the charged organic carrier to the charged antigen, by weight, is in the range of 5:1 to 0.5:1. Preferably, the ratio by weight is approximately 3:1 to 1:1, and more preferably the ratio by weight is 2:1.

Without limiting the present invention to any one theory or mode of action, it is thought that the complexing of the adjuvant with the antigen facilitates co-delivery of the adjuvant and the antigen to the same antigen presenting cell thereby facilitating the induction of immune responses which either would not occur or would not occur as effectively were these molecules not co-delivered. For example, the induction of some CD8+ cytotoxic T-lymphocyte responses are thought to occur where the adjuvant induces endosomal escape of the antigen in the antigen presenting cell. This necessarily requires co-delivery of the antigen and the adjuvant to the antigen presenting cell.

A further aspect of the present invention therefore relates to the use of the invention to induce an immune response in a mammal including, but not limited to, a humoral and/or cell mediated immune response.

Accordingly, another aspect of the present invention relates to a vaccine composition comprising as the active component an immunogenic complex comprising a charged organic carrier and a charged antigen, which organic carrier and antigen are electrostatically associated, and wherein the charged antigen is a polyprotein of Hepatitis C Virus (HCV) or a fragment thereof, or a fusion protein comprising said polyprotein or a fragment thereof, together with one or more pharmaceutically acceptable carriers and/or diluents.

Preferably, said organic carrier is an adjuvant, and even more preferably a saponin or a saponin complex. Preferably said saponin complex is ISCOMATRIX^{™}.

Preferably said organic carrier is negatively charged.

Vaccine compositions according to this aspect of the invention may be either prophylactic (i.e. used to prevent infection) or therapeutic (i.e. used to treat disease after infection). The vaccine compositions are conventionally administered parenterally, e.g. by injection, either subcutaneously, intramuscularly, or transdermally/transcutaneously. Additional formulations suitable for other modes of administration include oral and pulmonary formulations, suppositories, and transdermal applications. Dosage treatment may be a single dose schedule or a multiple dose schedule. The vaccine compositions may be administered in conjunction with other immunoregulatory agents.

These vaccine compositions comprise an immunogenic complex of the present invention in combination with one or more pharmaceutically acceptable carriers and/or diluents, such carriers include any carrier that does not itself induce the production of a response harmful to the individual receiving the composition. Suitable carriers are typically large, slowly metabolised macromolecules such as proteins, polysaccharides, polylactic acids, polyglycolic acids, polymeric amino acids, amino acid copolymers, lipid aggregates (such as oil droplets or liposomes), and inactive virus particles. Such carriers are well known to those of ordinary skill in the art. Additionally, these carriers may function as immunostimulating agents or adjuvants in addition to the adjuvant effect of the immunogenic complex itself. Furthermore, the antigen may be conjugated to a bacterial toxoid, such as a toxoid from diphtheria, tetanus, cholera, *H. pylori* and pathogens.

The vaccine compositions may also include further adjuvants to enhance effectiveness of the composition. Suitable adjuvants include, but are not limited to: (1) aluminum salts (alum), such as aluminum hydroxide, aluminum phosphate, aluminum sulfate, etc; (2) oil-in-water emulsion formulations (with or without other specific immunostimulating agents such as muramyl peptides (see below) or bacterial cell wall components), such as for example (a) MF59 (PCT Publ. No. WO 90/14837), containing 5% Squalene, 0.5% Tween 80, and 0.5% Span 85 (optionally containing various amounts of MTP-PE (see below), although not required) formulated into submicron particles, (b) SAF, containing 10% Squalene, 0.4% Tween 80, 5% pluronic-blocked polymer, and thr-MDP (see below) either microfluidised into a submicron emulsion or vortexed to generate a large particle size emulsion, and (c) Ribi^{™} adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL+CWS (Detox^{™}); (3) saponin adjuvants, such as Stimulon^{™} (Cambridge Bioscience, Worcester, MA); (4) Complete Freund's Adjuvant (CFA) and Incomplete Freund's Adjuvant (IFA); (5) cytokines, such as interleukins (e.g. IL-1, IL-2, IL-4, IL-5, IL-6, IL-7, IL-12, etc), interferons (e.g. gamma Interferon), macrophage colony stimulating favtor (M-CSF), tumor necrosis factor (TNF), etc.; (6) detoxified mutants of a bacterial ADP-ribosylating toxin such as a cholera toxin (CT), a pertussis toxin (PT), or an *E. coli* heat-labile toxin (LT), particularly LT-K63, LT-R72, CT-S109, PT-K9/G129; see, e.g. WO 93/13302 and WO 92/19265; (7) other substances that act as immunostimulating agents to enhance the effectiveness of the composition; and (8) microparticles with adsorbed macromolecules, as described in International Patent Application No. PCT/US99/17308. Alum and MF59 are preferred.

As mentioned above, suitable muramyl peptides include, but are not limited to, N-acetyl-muramyl-L-threonyl-D-isoglutamine (thr-MDP), N-acetyl-normauramyl-L-alanyl-D-isoglutamine (nor-MDP), N-acetylmuramyl-L-alanyl-D-isoglutaminyl-L-alanine-2-(1'-2'-dipalmitoyl-*sn*-glycero-3-hydroxyphosphoryloxy)-ethylamine (MTP-PE), etc.

The vaccine compositions typically will also contain diluents, such as water, saline, glycerol and ethanol. Additionally, auxiliary substances, such as wetting or emulsifying agents, pH buffering substances may be present in the compositions.

The forms suitable for injectable use include sterile aqueous solutions (where water soluble) or dispersions and sterile powders for the extemporaneous preparation of sterile injectable solutions or dispersions. They must be stable under the conditions of manufacture and storage and must be preserved against the contaminating action of microorganisms such as bacteria and fungi. The prevention of the action of microorganisms can be brought about by various antibacterial and antifungal agents, for example, parabens, chlorobutanol, phenol, sorbic acid, thimerosal. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compounds in the required amount in the appropriate solvent with various of the other ingredients enumerated above, as required, followed by filter sterilisation. Generally, dispersions are prepared by incorporating the various sterilised active ingredients into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

When the active ingredients are suitably protected they may be orally administered, for example, with an inert diluent or with an assimilable edible carrier, or they may be enclosed in hard or soft shell gelatin capsule, compressed into tablets, or incorporated directly with the food of the diet For oral therapeutic administration, the active compound may be incorporated with excipients and used in the form of ingestible tablets, buccal tablets, troches, capsules, elixirs, suspensions, syrups, wafers. Such compositions and preparations should contain at least 1% by weight of active compound. The percentage of the compositions and preparations may, of course, be varied and may conveniently be between about 5 to about 80% of the weight of the unit. The amount of active compound in such therapeutically useful compositions is such that a suitable dosage will be obtained. Preferred compositions or preparations according to the present invention are prepared so that an oral dosage unit form contains between about 0.1 µg and 2000 mg of active compound.

The tablets, troches, pills, capsules and the like may also contain the components as listed hereafter a binder such as gum, acacia, com starch or gelatin; excipients such as dicalcium phosphate; a disintegrating agent such as com starch, potato starch, alginic acid; a lubricant such as magnesium stearate; and a sweetening agent such as sucrose, lactose or saccharin may be added or a flavouring agent such as peppermint, oil of wintergreen, or cherry flavouring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavouring such as cherry or orange flavour. Of course, any material used in preparing any dosage unit form should be pharmaceutically pure and substantially non-toxic in the amounts employed. In addition, the active compound(s) may be incorporated into sustained-release preparations and formulations.

Without limiting the operation of the present invention in any way, the co-delivery of the immunogenic complex of the present invention is particularly useful for inducing an immune response and, in particular, a cytotoxic T-lymphocyte response to an antigen. Said immune response may be a specific (T cell and/or B cell) and/or non-specific immune response.

Accordingly, still another aspect of the present invention relates to a method of eliciting, inducing or otherwise facilitating, in a mammal, an immune response to an antigen, said method comprising administering to said mammal an effective amount of an immunogenic complex or a vaccine composition as hereinbefore described.

Preferably said immune response comprises a cytotoxic T-lymphocyte response.

It should be understood that the subject cytotoxic lymphocyte response may occur either in isolation or together with a helper T cell response, a humoral response or other specific or non-specific immune response.

A further aspect of the present invention relates to the use of the immunogenic complex of the invention in relation to the therapeutic and/or prophylactic treatment of disease conditions. Examples of disease conditions which can be treated in accordance with the method of the present invention include any disease condition which results from HCV infection.

Accordingly, yet another aspect of the present invention relates to a method of treating a disease condition in a mammal, said method comprising administering to said mammal an effective amount of an immunogenic complex or a vaccine composition as hereinbefore described, wherein administering said composition elicits, induces or otherwise facilitates an immune response which inhibits, halts, delays or prevents the onset or progression of the disease condition.

Direct delivery of the compositions will generally be accomplished by injection, either subcutaneously, intraperitoneally, intravenously or intramuscularly or delivered to the interstitial space of a tissue. The compositions can also be administered into a lesion. Other modes of administration include oral and pulmonary administration, suppositories, and transdermal or transcutaneous applications. Dosage treatment may be a single dose schedule or a multiple dose schedule.

An "effective amount" means an amount necessary at least partly to attain the desired immune response, or to delay the onset or inhibit progression or halt altogether, the onset or progression of a particular condition being treated. This amount varies depending upon the health and physical condition of the individual to be treated, the taxonomic group of individual to be treated, the capacity of the individual's immune system to synthesise antibodies, the degree of protection desired, the formulation of the vaccine, the assessment of the medical situation, and other relevant factors. It is expected that the amount will fall in a relatively broad range that can be determined through routine trials.

The term "mammal" includes humans, primates, livestock animals (eg. horses, cattle, sheep, pigs, donkeys), laboratory test animals (eg. mice, rats, rabbits, guinea pigs), companion animals (eg. dogs, cats) and captive wild animals (eg. kangaroos, deer, foxes). Preferably, the mammal is a human or laboratory test animal. Even more preferably, the mammal is a human.

The mammal undergoing treatment may be human or an animal in need of therapeutic or prophylactic treatment of a disease condition or a potential disease condition.

In yet another aspect the present invention relates to the use an immunogenic complex or vaccine composition as hereinbefore described in the manufacture of a medicament for inhibiting, halting, delaying or preventing the onset or progression of a disease condition.

Yet another aspect of the present invention relates to an agent for use in inhibiting, halting, delaying or preventing the onset or progression of a disease condition. Said agent comprising an immunogenic complex or vaccine composition as hereinbefore described.

Further features of the present invention are more fully described in the following non-limiting Examples.

Reference to "ISCOPREP^{™} 703" should be understood as a reference to a saponin preparation comprising from 50-90% by weight of Fraction A of Quil A and 50% to 10% by weight of Fraction C of Quil A. Fractions A and C are prepared from the lipophilic fraction of Quil A. Fractions "A" and "C", their method of preparation and the method of preparing ISCOPREP^{™} 703 are detailed in International Patent Publication No. WO96/11711.

Below are examples of specific embodiments for carrying out the present invention. The examples are offered for illustrative purposes only, and are not intended to limit the scope of the present invention in any way. Those of skill in the art will readily appreciate that the invention may be practiced in a variety of ways given the teaching of this disclosure.

Efforts have been made to ensure accuracy with respect to numbers used (e.g., amounts, temperatures), but some experimental error and deviation should, of course, be allowed for.

### Materials and Methods

### Animals.

Rhesus macaques (*Macaca mulatta)* were housed at Southwest Foundation for Biomedical Research (SFBR, San Antonio, TX). Studies were performed under the NIH Guidelines for Care and Use of Laboratory Animals (National Institute of Health. (1985) *Guide for the care and use of laboratory animals*. US department of Health and Human services. publication No 82-23. National Institute of Health, Bethesda, MD). Class I major histocompatibility complex (MHC) typing of the animals was performed as described (Urvater et al. (2000) J. Immunol. 164:1386.).

Female C57BU6 (H-2^{b}) mice were purchased from Charles River Laboratories and used between 8 and 10 weeks of age. Mice were housed in a pathogen free environment and were handled according to the international guidelines for experimentation with animals.

### Immunogens and Adjuvants.

The E. coli-derived full-length HCV-1a Core recombinant protein (aa: 1-191) was produced and purified under GMP conditions and is more than 98% pure. The recombinant HCV-1a E1 E2₈₀₉ protein was produced in CHO cells. This modified E1E2 protein contained amino acids 192 to 809. The recombinant NS35Core121 protein was produced in yeast cells. The adjuvant LTK63 is a genetically detoxified mutant of the heat-labile enterotoxin of Escherichia coli, in which the Serine at position 63 is replaced by a Lysine (Partidos et al. (1999) Immunol. Lett. 67:209.). The Core-ISCOM formulations were prepared by mixing the core protein with a preformed ISCOMATRIX^{™} (empty ISCOMs^{™}) utilizing ionic interactions to maximize association between the antigen and the adjuvant. ISCOMATRIX^{™} was prepared essentially by previously described methods except that diaflitration was used in place of dialysis (Coulter et al. (1998) Vaccine 16:1243). The oil-in-water adjuvant MF59 has been described (Ott et al. (1995) Pharm. Biotechnol. 6:277).

### Sucrose gradient analysis of Core-ISCOM

After formulation, preparations were purified on a sucrose gradient (10 to 50% w/v) and fractions analysed for ISCOMATRIX^{™} and protein to determine the amount of association. ISCOMATRIX^{™} was analysed using diphenylhexatriene (DPH) which fluoresces when associated with lipid. Briefly, DPH was dissolved at 1 mg/ml in acetone then diluted 1 in 50 in PBS pH7.2, then 50µl mixed with 50µl of each fraction in a microtitre plate. Following incubation for 150 mins at 20-25°C the plate was read in a fluorometer using excitation 355nm and emission 460nm. Protein was detected using Pierce Coomassie according to manufacturers instructions. Briefly, 50µl Coomassie solution was added to 50µl of each fraction in a microtitre plate. The plate was mixed and absorbance read at 595nm.

### Particle size analysis

Formulations were analysed for particle size by dynamic light scattering using a Nicomp Submicron Particle Sizer Model 370.

### Peptides and Vaccinia Viruses.

Peptides (15 or 20mer overlapping by 10 aa) spanning the entire length of the Core (aa: 1-191) protein of HCV-1a (Choo et al. (1991) Proc Natl Acad Sci USA 88:2451) were synthesized with free amine N-termini and free acid C-termini by Research Genetics (Huntsville, AL). The recombinant vaccinia virus (rVV) expressing Core and E1 (aa: 1-384; rWC/E1) and wild type VV (VVwt) have been described (Cooper et al. (1999) Immunity 10:439).

### Immunization.

Rhesus macaques were immunized under anesthesia. The first study was comprised of nine animals divided into three groups of three animals each. The first group (animals BB228, BB232 and DV036) were infected with 2 x 10⁸ plaque forming units (pfu) (1 x 10⁸ intradermally and 1 x 10⁸ by scarification) of rVVC/E1 at month 0. This group served as a positive control for CTL priming. Animals from the second group (AY921, BB231 and DV037) were immunized with 25µg of Core-ISCOM by intramuscular (IM) injection in the left quadriceps at month 0, 1, 2 and 6. Animals from the third group (AY922, BB227 and BB230) were immunized by IM injection with 200µg of HCV-Core protein adjuvanted with 200µg of LTK63 at month 0, 1, 2 and 6. For the second study, five animals (15860, 15861, 15862, 15863 and 15864) were immunized with 50µg of Core-ISCOM by IM injection in the left quadriceps at month 0, 1 and 2. Some Core-immunized animals (see Table I) also received 2 x 10⁸ pfu (1 x 10⁸ intradermally and 1 x 10⁸ by scarification) of rVVC/E1 nine or eleven weeks post their last vaccine immunization.

Mice (10 animals per group) were immunized in the tibialis anterior muscles (50µl per muscle) with 2µg per dose of recombinant E1 E2 protein alone or 2µg per dose of recombinant E1 E2 protein in the presence of MF59 (vol:vol), or 2µg per dose of recombinant E1 E2 protein + 2µg per dose of Core-ISCOM at weeks 0, 4 and 8.

### Cells and cell lines.

Peripheral blood was drawn from the femoral vein while the animals were under anesthesia. PBMCs were obtained after centrifugation over a Ficoll-hypaque gradient and cultured in 24-well dishes at 5 x 10⁶ cells per well. Of those cells, 1 x 10⁶ were sensitized with 10µM of a peptide pool (consisting of individual peptides) for one hour at 37°C, washed and added to the remaining 4 x 10⁶ untreated PBMCs in 2 ml of culture medium (RPMI 1640, 10% heat-inactivated fetal bovine serum, and 1 % antibiotics) supplemented with 10 ng/ml of IL-7 (R&D, Minneapolis, MN). After 48 hours, 5% (final) of interleukin-2 containing supernatant (T-STIM without PHA, Collaborative Biomedical Products, Bedford, MA) and 50 U/ml (final) of recombinant IL-2 (Chiron Corporation, Emeryville, CA) were added to the cultures. Cultures were fed every 3-4 days. After 10 days in culture, CD8+ T cells were isolated using anti-CD8 antibodies bound to magnetic beads (Dynal, Oslo, Norway) according to the manufacturer's instructions. Purified CD8+ cells (>93% pure as determined by flow cytometry) were cultured for another 2-3 days prior to being assayed for cytotoxic activity. Peptide-specific CD8+ lines were obtained by periodically restimulating these CD8+ T cells with autologous B-LCLs and peptide.

B-LCLs were derived from each animal using supernatants from the H. papio producer cell line S394.

### CTL assay.

Cytotoxic activity was assayed in a standard ⁵¹Cr release assay as described elsewhere (Paliard *et al.* (2000) *AIDS Res. Hum. Retroviruses* **16**:273). Briefly, B-LCLs were incubated with 10µM of peptides and 50µCi of ⁵¹Cr for 1 h, washed three times and plated at 5 x 10³ cells per well in a 96 well plate. Alternatively, B-LCLs were infected at multiplicity of infection (MOI) of 10:1 with rVVC/E1 or VVwt for 1 h, washed and cultured overnight prior to labeling with ⁵¹Cr. CD8+ cells were plated in duplicate at three different E:T ratio and incubated with target cells for 4 hours in the presence of 2 x 10⁵ per well of unlabeled target cells (cold targets), that were added to minimize lysis of B-LCLs by H. papio or endogenous virus (e.g. foamy virus)-specific CTLs. CTL responses were scored positive when percent specific lysis at the two highest E:T ratios were greater than or equal to the percent of lysis of control targets plus 10.

### Lymphoproliferation assay.

This assay has been described previously (Hong et al. (1997) J. Virol. 71:6427). Briefly, freshly isolated PBMCs were plated in triplicates at 2 x 10⁵ cells per well in 96 well round bottomed plates and cultured in the presence of 5µg/ml of recombinant Core protein or 0.05 µg/ml of *E. coli* control. Plates were pulsed with 1µCi per well of ³H-thymidine on day 5 and harvested 6-8 hours later. Results are presented as stimulation index (SI) calculated as (mean experimental cpm) / (mean cpm in the presence of the *E. coli* control). An Sl ≥3.0 was scored positive.

### FACS analysis.

Freshly isolated PBMCs or PBMCs that had been restimulated *in vitro* with a peptide were cultured in media alone or restimulated with 5 µg/ml of Core protein, 0.05 µg/ml of *E. coli* control, 5µg/ml of peptide, or VV-infected or peptide-sensitized autologous B-LCLs (1:1) for 12 hours in culture media containing 50 U/ml of rIL-2 (Chiron Corporation, Emeryville, CA) and 3 µM monensin (Pharmingen, San Diego, CA). Cells were stained according to Pharmingen's protocol for surface CD4 and CD8 with APC-conjugated anti-human CD4 and PerCP-conjugated anti-human CD8, and for intracellular IFN-γ and TNF-α with PE-conjugated anti-human IFN-γ and FITC-conjugated anti-human TNF-α. Antibodies were from Pharmingen and Becton-Dickinson (San Jose, CA). Cells were analyzed on a FACScalibur. Data files were analyzed using the CellQuest software.

### Cytokine ELISA.

Freshly isolated Rhesus macaque's PBMCs were restimulated with peptides encompassing the whole Core protein. Levels of Rhesus monkey IL-2, IL-5, IL-10 and IFN-γ present in 48 hours cell-free culture supernatants were determined by specific ELISA (U-Cytech, Utrecht, The Netherlands) following the manufacturers' specification.

### HCV antibodies.

Serum levels of HCV Core and HCV E2 antibodies were quantified by ELISA as described (Chien et al. (1992) Proc Natl Acad Sci USA 89:10011). Serum levels of antibodies inhibiting the binding of E2 to the putative HCV receptor CD81 (Pileri et al. (1998) Science 282:938) were determined by immunoassay.

### Example 1

### Sucrose Gradient Analysis of Core-ISCOM

Core Protein was found in fractions 5 to 11 (Figure 1A) whilst ISCOMATRIX^{®} was found in fractions 9 to 13 (Figure 1B). The Core-ISCOM was found with in fractions 14-17 with both the ISCOM^{™} and the protein peaks overlapping, which indicates association (Figure 1 C).

### Example 2

### Stability of Core-ISCOM

The stability of the Core-ISCOM™ formulation was evaluated for 11 months. Both the particle size and association remained consistent for at least 11 months at 2-8°C (Table I).

### Example 3

### Priming of Core-specific CTLs in Vaccinated Animals

As explained above, two different prototype vaccines (Core-ISCOM and Core + LTK63) aimed at eliciting HCV-Core-specific CTLs were each administered to three HCV-naïve Rhesus macaques (see Table II for animal assignment, dosage and immunization schedule). Since it was unknown whether Rhesus macaques' MHC class I molecules can bind and present HCV-Core -derived peptides and whether the positively selected CD8+ T cell repertoire in these animals can recognize such MHC class I - Core-derived peptide complexes, three additional animals were inoculated with 2 x 10⁸ pfu of rVVC/E1 to serve as positive controls (Table II).

None of the nine animals had any detectable CTLs at the time of immunization (week 0; Table III and data not shown). This confirmed that these animals had not been previously exposed to HCV Core and that restimulation of PBMCs under the conditions described in Material and Methods did not result in the priming of primary CTL responses *in vitro.* Two weeks post rVVC/E1 infection, two (BB232 and DV036) out of the three animals had detectable CTLs against Core peptides pool 4 (aa: 121-170) and pool 3 (aa: 81-130), respectively (Table III). By deconvoluting these peptide pools, it was determined that BB232's CTLs recognized the epitopic peptide 121-135 and that DV036's CTLs recognized peptide 86-100. The presence of 121-135 and 86-100-specific CTLs in these rVVC/E1-inoculated animals indicated that both peptides were naturally processed. No CTL responses were detectable in the other rVVC/E1-inoculated animal (BB228). This indicated that Core-specific CTLs can be elicited in at least some Rhesus monkeys. Out of the three animals that received Core adjuvanted with LTK63 (Table II), only one animal (BB230) showed a CTL response against Core. This response, directed against pool 2 (aa: 41-90), was transient, however, as it was detectable two weeks post 3^{rd} immunization, but was undetectable six weeks post 3^{rd} immunization. Furthermore, these CTLs were not boosted by a 4^{th} immunization (Table III) and the individual peptide recognized could not be identified. Two out of the three Core-ISCOM-immunized animals (AY921 and BB231; Table II) did not mount a detectable Core-specific CTL response. In contrast, in the other Core-ISCOM -immunized animal (DV037), CTLs recognizing pool 4 (aa: 121-170) were detectable as early as two weeks post 2^{nd} immunization. This response was directed against the epitopic peptide aa: 121-135 and was also present post 3^{rd} and post 4^{th} immunization (Table III).

In contrast to animals that received the Core + LTK63 prototype vaccine, animals immunized with the Core-ISCOM prototype vaccine had detectable CTLs post second immunization and these CTLs were also present post 3^{rd} and 4^{th} immunization (Table III). Thus, this latter vaccine seemed more potent at eliciting CTL responses in Rhesus macaques than the former one. However, this formulation primed Core-specific CTLs in only one out of the three animals. This might be due to the fact that the MHC class I molecules of the non-responding animals were unable to bind and present peptides derived from this relatively small protein (191 aa). To test this hypothesis, CTL lines specific for peptide 121-135 and 86-100 were established from responding animals. As shown in Figure 2A, the peptide 121-135-specific CTL line lysed peptide-sensitized B-LCLs derived from DV037, but did not kill peptide 121-135-sensitized B-LCLs from the two non-responding animals (AY921 and BB231). Similarly, B-LCLs derived from DV036 but not AY921 or BB231 were able to present peptide 86-100 to CD8+ CTLs (Figure 2B). These data indicated that AY921's and BB231's MHC class I molecules could not present these peptides to CD8+ T cells, and suggested that MHC class I haplotypes determined whether Rhesus monkeys could mount a CTL response to HCV-Core.

Since different MHC class I alleles can bind and present different sets of peptides, these data did not rule out that the Core-ISCOM formulation was not sub-optimal, i.e. it remained possible that these animals could mount a Core-specific CTL response directed against peptides other than 86-100 and 121-135. To address this possibility, AY921 and BB231 were challenged with 2 x 10⁸ pfu of rVVC/E1 eleven weeks after their 4^{th} immunization with Core-ISCOM. As opposed to BB232 and DV036 which had Core-specific CTLs two weeks post rVVC/E1 infection (Table III), neither AY921 nor BB231 had any detectable CTLs post rVVC/E1 inoculation. This strongly suggested that the absence of detectable Core-specific CTLs after Core-ISCOM immunization of these animals was not due to a sub-optimal vaccine formulation, but to an intrinsic inability of these animal to mount such a response, presumably a consequence of their MHC class I haplotype.

To investigate whether immunization with Core-ISCOM induced long-lived CTLs, we monitored DV037 for up to 51 weeks (1 year) after its 4^{th} immunization. Peptide 121-135-specific CTLs were detected 10, 15, 31, 38, 45 and 51 weeks post last immunization (Figure 3A). In contrast, the 121-135-specific CTL response primed by rVVC/E1 in BB232 was barely detectable 14 weeks post vaccination and was undetectable 18 weeks post vaccination (Figure 3B). Similarly, the 86-100-specific CTLs primed in DV036 by rVVC/E1 vaccination became undetectable 14 weeks post vaccination.

In an effort to quantify the number of peptide 121-135-specific CTLs present in DV037 1 year after it had received its last boost, the animal's PBMCs were restimulated *ex vivo* with 121-135 and the percent of specific CD8+ T cells was assessed by intracellular staining for IFN-γ and TNF-α. As illustrated in Figure 3C (left panels), 121-135-specific CTLs represented 0.49% of the peripheral CD8+ T cells (or 490 cells per 10⁵ CD8+ T cells) secreted IFN-γ and/or TNF-α after *ex vivo* peptide stimulation for 12 hours. In contrast, after *in vitro* restimulation with peptide 121-135, 71 % of these CD8+ T cells were specific for this peptide as determined by their abilities to secrete IFN-γ and/or TNF-α (Figure 3C, right panels).

### Example 4

### Characterization of Cellular and Humoral Immune Responses in Rhesus Monkeys Immunized with Core-ISCOM

Although only one out of three Core-ISCOM immunized animals had detectable CTLs, the fact that in the responding animal Core-specific CTLs were detected after only two immunizations (Table III) and were long-lived (Figure 3A) formed the basis to immunize five more animals (15860-4) with Core-ISCOM (see Table II for dosage and immunization schedule). All animals were naïve at the time of vaccination. In this study, the priming of both Core-specific CTLs and Core-specific CD4+ T cells and antibodies were monitored.

None of the animals had any detectable Core-specific CD4+ or CD8+ T cells at the time of immunization (week 0, Table IV). Core-specific CD4+ T cells, as determined by lymphoproliferation assay, were detected in all animals except 15861 after the second immunization, but this animal had a detectable CD4+ response after the third immunization (Table IV). For animals 15862 and 15863, it is unlikely that the low SI observed after the 3^{rd} immunization (Table IV) was due to the absence of a CD4+ T cell response as a strong proliferation was observed in these animals and at this particular time point for the *E. coli* control (see below). None of the animals had antibodies against Core prior to immunization. However, all animals had seroconverted to Core after two immunizations, and the level of antibodies against Core was boosted by a third immunization (Figure 4). Notably, the mean Core antibody titer among these animals was comparable after two immunization (1,931) and higher after three immunization (4,566) (Figure 4) to that present in the serum of chronically infected patients with an unusually high anti-Core antibody titer (2,358; not shown) run in the same assay.

To investigate whether Core-ISCOM elicited a Th1 or Th2-type response in these monkeys, freshly isolated PBMCs prior to vaccination as well as two weeks post 2^{nd} and 3^{rd} immunizations were tested for their capacity to produce cytokines at 48h in response to stimulation with Core peptides spanning the entire length of the Core protein. As shown in Figures 5A and 5B, a significant increase in Th1 cytokines (IFN-γ and IL-2) was observed post immunization in all animals, although the magnitude of response observed for 15860, 15861 and 15862 was lower than that observed for animals 15863 and 15864. Similarity, an increase in Th2-type cytokines (IL-5 and IL-10) was observed in all animals following vaccination, with the highest amount of IL-5 and IL-10 also detected in 15863 and 15864 (Figures 5C and 5D). Although the amount of secreted Th2-type cytokines were lower than that detected for Th1-type cytokines, these data indicated that Core-ISCOM induced a Th0-like type response in Rhesus monkeys.

After two and three immunizations, three animals (15862, 15863 and 15864) had detectable CTL responses directed against pool B (aa: 60-140), while the two others (15860 and 15861) did not (Table IV). This CTL response was directed against peptide 86-100 for animal 15864 and against peptide 121-135 for animals 15862 and 15863. Since 86-100 and 121-135-specific CTLs were also primed in DV036 (86-100), DV037 (121-135) and BB 232 (121-135) (Table III), it was of importance to determined whether all 86-100 and 121-135-specific CTLs were respectively restricted by a single MHC class I allele. The 86-100-specific CTL line derived from animal 15864 efficiently lysed peptide 86-100-sensitized B-LCLs derived from 15864 but not peptide-sensitized B-LCLs derived from animal DV036, indicating that a different (unidentified) MHC class I allele presented this peptide to CTL (Figure 6A and Table II). In contrast, the CTLs specific for peptide 121-135 from animal 15862, 15863, BB232 and DV037 were restricted by a single, yet unidentified, MHC class I allele shared by all these animals (Figure 6B and Table II). These data also indicated that the MHC class I molecules of 15860 and 15861 could not present either peptide (86-100 and 121-135) to specific CTLs (Figures 6A and 6B). As observed in the first study, rVVC/E1 infection of the two non-responding animals (15860 and 15861) nine weeks post 3^{rd} immunization with Core-ISCOM did not lead to the priming of Core-specific CTLs in these animals. Taken together, this suggested, once again, that the MHC class I haplotype of the animals dictated whether they could mount Core-specific CTLs.

In an effort to quantitate the number of Core-specific CD8+ and CD4+ T cells primed in the animals described above, freshly isolated PBMCs were stained for intracellular INF-γ and TNF-α after *ex vivo* restimulation. The CD8+ T cell responses to naturally processed peptides were quantified after ex *vivo* restimulation with autologous B-LCLs infected with rVVC/E1 or VVwt, as a control. The CD4+ T cell responses to naturally processed peptides were quantified after *ex vivo* restimulation with the recombinant Core protein or an *E. coli* control. Intracellular staining responses revealed that while none of the animals had detectable Core-specific CD8+ T cells at the time of immunization, between 0.30 and 0.71% of 15862, 15863 and 15864's peripheral CD8+ T cells were specific for naturally processed Core-derived peptide(s) after 2 immunizations (Figure 7A). The number of specific CTLs was, however, not increased after the third immunization, as judged by intracellular staining responses. Notably, no CD8+ T cells secreting IFN-γ and/or TNF-α in response to Core were detected in the two animals (15860 and 15861) for whom no Core-specific CTL activity was observed by ⁵¹Cr release assay (Figure 7A and Table IV). Quantification of Core-specific CD4+ T cells confirmed the data obtained by lymphoproliferation assay (Table IV) in that between 0.32 and 2.21% of CD4+ T cells from all five animals were specific for naturally-processed Core peptides (Figure 7B). Furthermore, the fact that 0.53 and 0.28% of CD4+ T cells from animals 15862 and 15863 were positive for cytokines after the 3^{rd} immunization (Figure 7B), strongly suggested that the negative SI observed for this time point (Table IV) was indeed a 'false negative', most likely due to the high proliferation observed in response to the *E. coli* control. This suggested that intracellular staining for IFN-γ and TNF-α is a more sensitive assay than lymphoproliferation to assess antigen-specific CD4+ T cell responses. Indeed, for animal 15861, no CD4+ T cells were detected by lymphoproliferation 2 weeks post 2^{nd} immunization (Table IV). In contrast, Core-specific CD4+ T cells were detected in this animal (2 weeks post 2^{nd}) by intracellular staining for IFN-γ and TNF-α (Figure 7B).

### Example 5

### The Use of Core-ISCOMs as Adjuvants for HCV Polypeptides

Because vaccination with recombinant HCV envelope proteins and adjuvant can, at least in some instances, influence the outcome of infection and disease (Choo et al. (1994) Proc. Natl. Acad. Sci. USA 91:1294), we investigated whether the Core-ISCOM above could serve as an adjuvant for other HCV polypeptides, such as the heterodimeric envelope protein E1 E2. To that end, mice (10 animals per group) were immunized with 2µg of soluble E 1 E2 protein alone, or 2µg of soluble E 1 E2 in the presence of the adjuvant MF59, or in the presence of 2µg of Core-ISCOM. As shown in Figure 8, mice immunized with E1 E2 alone had no significant anti-E2 antibody titer. In contrast, mice immunized with E1E2 + Core-ISCOM had a significant anti-E2 antibody titer after three immunizations, and these titers were comparable to those observed in mice immunized with E1 E2 + MF59. Furthermore, the 'quality' of antibody elicited in mice immunized by E1 E2 + MF59 and E1 E2 + Core-ISCOM appeared to be comparable with the antibody titers that could inhibit the binding of HCV-1a E2 to the HCV putative receptor CD81 in both groups of mice (Figure 9).

The above examples demonstrate that vaccination with HCV polypeptide ISCOMs are able to prime strong HCV polypeptide-specific CD8+ and CD4+ T cells as well as anti-HCV polypeptide antibodies. Furthermore these ISCOM formulations are able to serve as adjuvants to elicit antibodies against other HCV proteins. Thus, HCV ISCOMs may prevent the establishment of chronicity, and/or increase the response rate to anti-viral therapy.

### Example 6

### Sucrose Gradient Analysis of NS35Core121-ISCOM

The NS35Core121 protein was found in a broad peak across the gradient (Figure 10A). The NS35Core121 ISCOM the protein was essentially found in fractions 15 to 20 which corresponded to an ISCOMATRIX^{™} peak indicating association has occurred (Figure 10B). Interestingly an ISCOMATRIX^{™} was also found in fractions 5 to 10 which indicates there was a proportion of the ISCOMATRIX^{™} with no protein associated.

Accordingly, novel HCV/ISCOM compositions and methods of using the same have been disclosed.

**Table I - Stability of Core-ISCOM™ Formulations**

| **Time months** | **Particle size** | **Sucrose gradient** |
|---|---|---|
| 0 | 1.5 | >90% associated |
| 1 | 1.7 | >90% associated |
| 11 | 1.8 | >90% associated |

**Table II - Summary of Immunization and MHC-I type.**

| **Animal Number** | **MHC class I Tping (Mamu A* & B*)^{a}** | **Immunogen** | **Dose (Route)** | **Immunization schedule (weeks)** |
|---|---|---|---|---|
| **First Study** | | | | |
| BB228 | A*08; B*17 | | | |
| BB232 | B*03 | rVVC/E1 | 1x10⁸ pfu | 0 |
| DV036 | B*03 | | (scarification) | |
| | | | 1x10⁸ pfu (ID) | |
| AY921^{b} | A*08; B*03 | | | |
| BB231^{b} | A*01; A*02 | HCV Core ISCOM^{™} | 25 µg (IM) | |
| DV037 | B*03; B*04 | | | 0, 4, 8, 27 |
| AY922 | B*01 | | | |
| BB227 | A*02 | HCV Core | 200 µg (IM) | |
| BB230 | A*08; B*03 | Adjuvant: LTK63 | 200 µg (IM) | 0, 4, 8, 27 |

| **Second Study** | | | | |
|---|---|---|---|---|
| 15860^{b} | B*01; B*03 | | | |
| 15861^{b} | B*03 | HCV Core ISCOM^{™} | 50 µg (IM) | 0, 4, 8 |
| 15862 | B*03 | | | |
| 15863 | - | | | |
| 15864 | B*01 | | | |

| | | | | |
|---|---|---|---|---|
| ^{a} Mamu alleles tested for: A*01, *02, *08 and 11; B01, *03, *04 and *17. ^{b} These animals also received 2 x 10⁸ pfu or rVVC/E1 nine to eleven weeks post last immunization. ID: Intradermal; IM: intramuscular. | | | | |

**Table III - Priming of Core-specific CTLs in Rhesus macaques.**

| Percent Specific Lysis^{a} | | | | | | | | | |
|---|---|---|---|---|---|---|---|---|---|
| | E:T Ratio | DV037 | | BB230 | | BB232 | | DV036 | |
| | | (Core-ISCOMS) | | (Core+LTK63) | | (rVVC/E1) | | (rWC/E1) | |
| | | Core pool 4 | | Core Pool 2 | | Core pool 4 | | Core pool 3 | |
| | | (aa: 121-170) | | (aa 41-90) | | (aa:121-170) | | (aa: 81-130) | |
| Week | | Hm^{b} | Ht^{b} | Hm^{b} | Ht^{b} | Hm^{b} | Ht^{b} | Hm ^{b} | Ht^{b} |
| 0 | 40:1 | <1 | <1 | 11 | <1 | 9 | <1 | 8 | 12 |
| (pre) | 13:1 | 10 | <1 | <1 | <1 | 7 | <1 | <1 | 4 |
| | 4:1 | 2 | <1 | <1 | <1 | 7 | <1 | <1 | <1 |
| 2 | 40:1 | NT | NT | NT | NT | 44^{c} | <1 | 24^{d} | 5 |
| (2w post 1^{st}) | 13:1 | NT | NT | NT | NT | 25 | <1 | 15 | <1 |
| | 4:1 | NT | NT | NT | NT | 13 | <1 | 6 | <1 |
| 6 | 40:1 | 20 | <1 | 17 | 6 | N/A | N/A | N/A | N/A |
| (2w post 2^{nd}) | 13:1 | 10 | <1 | <1 | 13 | N/A | N/A | N/A | N/A |
| | 4:1 | 5 | <1 | <1 | 10 | N/A | N/A | N/A | N/A |
| 10 | 40:1 | 20 | <1 | 49 | 9 | N/A | N/A | N/A | N/A |
| (2w post 3^{rd}) | 13:1 | 12 | <1 | 23 | <1 | N/A | N/A | N/A | N/A |
| | 4:1 | <1 | <1 | 14 | <1 | N/A | N/A | N/A | N/A |
| 14 | 40:1 | 30^{c} | 5 | 40 | 30 | N/A | N/A | N/A | N/A |
| (6w post 3^{rd}) | 13:1 | 16 | <1 | 14 | 15 | N/A | N/A | N/A | N/A |
| | 4:1 | 12 | <1 | 9 | 10 | N/A | N/A | N/A | N/A |
| 29 | 40:1 | 50 | 2 | 17 | <1 | N/A | N/A | N/A | N/A |
| (2w post 4^{th}) | 13:1 | 35 | 3 | <1 | <1 | N/A | N/A | N/A | N/A |
| | 4:1 | 28 | 2 | <1 | <1 | N/A | N/A | N/A | N/A |

| | | | | | | | | | |
|---|---|---|---|---|---|---|---|---|---|
| NT: Not Tested; N/A: Not applicable. ^{a} Percent Specific Lysis is only shown for animals with detectable CTL activity and for the peptide pools against which such CTL activity was detected. ^{b} CT- activity of CD8+ T cells restimulated *in vitro* with a peptide pool (1 through 5) was tested against autologous B-LCLs sensitised with the same peptide pool (Hm) or an irrelevant peptide pool (Ht). ^{c} The epitopic peptide recognised is aa: 121-135 (KVIDTLTCGFADLMG). ^{d} The epitopic peptide recognised is aa: 86-100 (YGNEGCGWAGWLLSP). | | | | | | | | | |

**Table IV - Priming of Core-specific CD8+ and CT4+ in Rhesus macaques immunized with core-ISCOMs.**

| | | Animal #15860 | | | Animal #15861 | | | Animal #15862 | | | Animal #15863 | | | Animal #15864 | | |
|---|---|---|---|---|---|---|---|---|---|---|---|---|---|---|---|---|
| Week | E:T Ratio | CD8 resp. (% Lysis)^{a} | | CD4+ resp. (SI)^{b} | CD8 resp. (% Lysis)^{a} | | CD4+ resp. (SI)^{b} | CD8 resp. (% Lysis)^{a} | | CD4+ resp. (SI)^{b} | CD8 resp. (% Lysis)^{a} | | CD4+ resp. (SI)^{b} | CD8 resp. (% Lysis)^{a} | | CD4+ resp. (SI)^{b} |
| | | Core pool B | | | Core pool B | | | Core pool B | | | Core pool B | | | Core pool B | | |
| | | (aa: 61-140) | | | (aa: 61-140) | | | (aa: 61-140) | | | (aa: 61-140) | | | (aa: 61-140) | | |
| | | Hm | Ht | | Hm | Ht | | Hm | Ht | | Hm | Ht | | Hm | Ht | |
| 0 | 40:1 | 8 | 13 | | 4 | 1 | | 14 | 14 | | 11 | 5 | | 13 | 3 | |
| (pre | 13:1 | 2 | 11 | 1.7 | <1 | <1 | 0.4 | 6 | 8 | 0.9 | <1 | 4 | 1.2 | <1 | 13 | 0.8 |
| | 4:1 | 2 | 5 | | <1 | <1 | | 3 | <1 | | 1 | 3 | | <1 | <1 | |
| 6 | 40:1 | 8 | 3 | | 7 | <1 | | 31^{c} | 8 | | 34^{c} | 9 | | 42^{d} | 18 | |
| (2w post | 13:1 | <1 | 2 | 5.5 | <1 | <1 | 1.9 | 16 | 6 | 5.0 | 16 | 6 | 3.5 | 20 | 4 | 3.8 |
| 2^{nd}) | 4:1 | <1 | <1 | | <1 | < | | 4 | <1 | | 8 | 1 | | 12 | 3 | |
| 10 | 40:1 | <1 | 3 | | 13 | 13 | | 31 | 5 | | 29 | 1 | | 79 | 10 | |
| (2w post | 13:1 | <1 | 12 | 7.1 | 5 | 7 | 4.5 | 14 | 4 | 2.1^{e} | 17 | <1 | 1.1^{e} | 60 | 11 | 8.8 |
| 3^{rd}) | 4:1 | <1 | <1 | | <1 | 2 | | 6 | <1 | | 8 | <1 | | 33 | 11 | |

| | | | | | | | | | | | | | | | | |
|---|---|---|---|---|---|---|---|---|---|---|---|---|---|---|---|---|
| ^{a} CTL activity of CD8+ T cells restimulated *in vitro* with a peptide pool (A through C) was tested against autologous B-LCLs sensitized with the same peptide pool (Hm) or an irrelevant peptide pool (H1). Percent Specific Lysis is only shown for pool B since no CTL activity was detected in any animals for pool A (aa: 1-70) or pool C )aa: 131-191). ^{b} CD4+ response was determined by lymphoproliferation assay as described in Materials and Methods. A stimulation index (SI) of 3.0 or greater was scored positive. ^{c} epitope recognised was aa: 121-130 (KVIDTLTCGFADLMG). ^{d} epitope recognised was aa: 86-100 (YGNEGCGWAGWLLSP). ^{e} High proliferation to *E. coli* control. | | | | | | | | | | | | | | | | |

## Claims

1. An immunogenic complex comprising a negatively charged organic complex and a charged antigen, which organic complex and antigen are electrostatically associated, wherein the organic complex is a negatively charged adjuvant and comprises a saponin and cholesterol and wherein the charged antigen comprises (i) one or more immunogenic polypeptides from a region of Hepatitis C Virus (HCV) selected from Core, NS3, NS4a, NS4b, NS5a and NS5b, or (ii) a fusion protein comprising an immunogenic polypeptide from the Core region of HCV and a second immunogenic polypeptide selected from the E1, E2, NS3, NS4a, NS4b, NS5a and NS5b regions of HCV.

2. The immunogenic complex according to claim 1 wherein the degree of negative charge of the organic complex is increased by adding a negatively charged detergent or lipid.

3. The immunogenic complex according to claim 1 or 2 wherein said organic complex further comprises a phospholipid.

4. The immunogenic complex according to claim 3 wherein said phospholipid is a phosphoglyceride.

5. The immunogenic complex according to claim 4 wherein the phosphoglyceride is selected from phosphatidyl inositol, phosphatidyl glycerol, phosphatidic acid and cardiolipin.

6. The immunogenic complex according to claim 3 wherein said phospholipid is lipid A.

7. The immunogenic complex according to claim 6 wherein the lipid A is selected from diphosphoryl lipid A and monophosphoryl lipid A.

8. The immunogenic complex according to any one of the preceding claims wherein said complex induces a cytotoxic T-lymphocyte response.

9. A vaccine composition comprising as the active component an immunogenic complex as defined in any one of the preceding claims together with one or more pharmaceutically acceptable carrier and/or diluents.

10. Use of an immunogenic complex according to any one of claims 1 to 8 or a vaccine composition according to claim 9 in the manufacture of a medicament for use in a method of treating or preventing an HCV infection in a mammal by eliciting, inducing or otherwise facilitating, an immune response to an HCV antigen.

11. Use according to claim 10, wherein said immune response comprises a cytotoxic T-lymphocyte response.

12. Use according to claim 10 wherein said immune response inhibits, halts, delays or prevents the onset or progression of a disease condition which results from an HCV infection.

13. An immunogenic complex according to any one of claims 1 to 8 or a vaccine composition according to claim 9 for use in a method of treating the human or animal body by therapy.

## Patentansprüche

1. Immunogener Komplex, der einen negativ geladenen organischen Komplex und ein geladenes Antigen umfasst, wobei der organische Komplex und das Antigen elektrostatisch assoziiert sind, wobei der organische Komplex ein negativ geladenes Adjuvans ist und ein Saponin und Cholesterin umfasst und wobei das geladene Antigen (i) ein oder mehrere immunogene Polypeptide von einer Region des Hepatitis-C-Virus (HCV), die aus Core, NS3, NS4a, NS4b, NS5a und NS5b ausgewählt ist, oder (ii) ein Fusionsprotein, das ein immunogenes Polypeptid aus der Core-Region von HCV und ein zweites immunogenes Polypeptid, das aus den E1-, E2-, NS3-, NS4a-, NS4b-, NS5a- und NS5b-Regionen von HCV ausgewählt ist, umfasst, umfasst.

2. Immunogener Komplex nach Anspruch 1, wobei der Grad der negativen Ladung des organischen Komplexes durch Hinzufügen eines negativ geladenen Detergens oder Lipids erhöht ist.

3. Immunogener Komplex nach Anspruch 1 oder 2, wobei der organische Komplex ferner ein Phospholipid umfasst.

4. Immunogener Komplex nach Anspruch 3, wobei das Phospholipid ein Phosphoglycerid ist.

5. Immunogener Komplex nach Anspruch 4, wobei das Phosphoglycerid aus Phosphatidylinosit, Phosphatidylglycerin, Phosphatitsäure und Cardiolipin ausgewählt ist.

6. Immunogener Komplex nach Anspruch 3, wobei das Phospholipid Lipid A ist.

7. Immunogener Komplex nach Anspruch 6, wobei das Lipid A aus Diphosphoryllipid A und Monophosphoryllipid A ausgewählt ist.

8. Immunogener Komplex nach einem der vorhergehenden Ansprüche, wobei der Komplex eine cytotoxische T-Lymphozytenantwort induziert.

9. Vaccinzusammensetzung, die als aktive Komponente einen immunogenen Komplex gemäß der Definition in einem der vorhergehenden Ansprüche zusammen mit einem oder mehreren pharmazeutisch akzeptablen Trägern und/oder Verdünnungsmitteln umfasst.

10. Verwendung eines immunogenen Komplexes nach einem der Ansprüche 1 bis 8 oder Vaccinzusammensetzung nach Anspruch 9 bei der Herstellung eines Medikaments zur Verwendung in einem Verfahren zur Behandlung oder Prävention einer HCV-Infektion in einem Säuger durch Auslösen, Induzieren oder in anderer Weise Ermöglichen einer Immunantwort auf ein HCV-Antigen.

11. Verwendung nach Anspruch 10, wobei die Immunantwort eine cytotoxische C-Lymphozytenantwort umfasst.

12. Verwendung nach Anspruch 10, wobei die Immunantwort den Ausbruch oder die Weiterentwicklung eines Krankheitszustands, der von einer HCV-Infektion herrührt, hemmt, stoppt, verzögert oder verhindert.

13. Immunogener Komplex nach einem der Ansprüche 1 bis 8 oder Vaccinzusammensetzung nach Anspruch 9 zur Verwendung in einem Verfahren zur Behandlung des menschlichen oder Tierkörpers durch eine Therapie.

## Revendications

1. Complexe immunogène comprenant un complexe organique négativement chargé et un antigène chargé, ce complexe organique et cet antigène étant en association électrostatique, dans lequel le complexe organique est un adjuvant négativement chargé et comprend une saponine et du cholestérol et dans lequel l'antigène chargé comprend (i) un ou plusieurs polypeptides immunogènes provenant d'une région du virus de l'hépatite C (HCV) choisie parmi Core, NS3, NS4a, NS4b, NS5a et NS5b, ou (ii) une protéine de fusion comprenant un polypeptide immunogène de la région Core de HCV et un second polypeptide immunogène choisi parmi les régions E1, E2, NS3, NS4a, NS4b, NS5a et NS5b de HCV.

2. Complexe immunogène selon la revendication 1, dans lequel le degré de charge négative du complexe organique est accrue par addition d'un détergent ou d'un lipide négativement chargé.

3. Complexe immunogène selon la revendication 1 ou 2, dans lequel ledit complexe organique comprend en outre un phospholipide.

4. Complexe immunogène selon la revendication 3, dans lequel ledit phospholipide est un phosphoglycéride.

5. Complexe immunogène selon la revendication 4, dans lequel le phosphoglycéride est choisi parmi les phosphatidylinositol, phosphatidylglycérol, acide phosphatidique et cardiolipine.

6. Complexe immunogène selon la revendication 3, dans lequel ledit phospholipide est le lipide A.

7. Complexe immunogène selon la revendication 6, dans lequel le lipide A est choisi parmi le disphosphoryl-lipide A et le monophosphoryl-lipide A.

8. Complexe immunogène selon l'une quelconque des revendications précédentes, ledit complexe induisant une réponse des lymphocytes T cytotoxiques.

9. Composition vaccinale comprenant comme ingrédient actif un complexe immunogène défini dans l'une quelconque des revendications précédentes avec un ou plusieurs supports et/ou diluants pharmaceutiquement acceptables.

10. Utilisation d'un complexe immunogène selon l'une quelconque des revendications 1 à 8 ou d'une composition vaccinale selon la revendication 9 dans la fabrication d'un médicament pour une utilisation dans un procédé de traitement ou de prévention d'une infection par HCV chez un mammifère en provoquant, induisant ou facilitant d'une autre manière, une réponse immunitaire à un antigène de HCV.

11. Utilisation selon la revendication 10, dans laquelle ladite réponse immunitaire comprend une réponse des lymphocytes T cytotoxiques.

12. Utilisation selon la revendication 10, dans laquelle ladite réponse immunitaire inhibe, arrête, retarde ou prévient le déclenchement ou l'évolution d'une condition pathologique qui résulte d'une infection par HCV.

13. Complexe immunogène selon l'une quelconque des revendications 1 à 8 ou composition vaccinale selon la revendication 9 pour une utilisation dans un procédé de traitement de l'organisme humain ou animal par thérapie.
